# EUROPÄISCHE PATENTSCHRIFT

(11) **EP 3 586 032 B1**
(45) Veröffentlichungstag und Bekanntmachung des Hinweises auf die Patenterteilung: **21.04.2021**
(21) Anmeldenummer: 18711490.5
(22) Anmeldetag: 26.02.2018
(51) Int. Cl.: F16F 9/53, F16F 9/14, F16F 9/06

(54) **PROTHESENEINRICHTUNG MIT EINEM DREHDÄMPFER**
PROSTHETIC DEVICE HAVING A ROTARY DAMPER
DISPOSITIF PROTHÉTIQUE COMPRENANT UN AMORTISSEUR ROTATIF

(30) Priorität: 24.02.2017 DE 102017103809
(43) Veröffentlichungstag der Anmeldung: 01.01.2020
(73) Patentinhaber: INVENTUS Engineering GmbH, 6771 St. Anton i.M. (AT)
(72) Erfinder: BATTLOGG, Stefan, 6771 St. Anton i.M. (AT)
(74) Vertreter: BSB Patentanwälte Schütte & Engelen Partnerschaft mbB
(86) Internationale Anmeldenummer: PCT/EP2018/054685
(87) Internationale Veröffentlichungsnummer: WO 2018/154117

(56) Entgegenhaltungen:
- EP-A1- 1 531 283
- EP-A1- 2 875 255
- DE-A1-102015 104 927
- JP-A- H08 177 939
- JP-A- 2009 287 639

## Beschreibung

Die vorliegende Erfindung betrifft eine Protheseneinrichtung mit einem Drehdämpfer, wobei der Drehdämpfer ein Gehäuse und eine drehbar daran aufgenommene Dämpferwelle und eine Verdrängereinrichtung in dem Gehäuse umfasst. In dem Gehäuse ist ein Dämpfervolumen mit einem magnetorheologischen Fluid als Arbeitsfluid vorgesehen, um eine Dämpfung der Dreh- oder Schwenkbewegung der Dämpferwelle relativ zu dem Gehäuse zu beeinflussen.

Die WO 2014/013435 A1 offenbart einen Steuerkopf für ein Motorrad, dessen Drehbewegung über einen magnetorheologischen Dämpfer gedämpft werden kann.

Die JP 2009-287639 A zeigt einen einen Rotationsdämpfer, der zur Verwendung in einem vierrädrigen Fahrzeug geeignet ist.

Aus der DE 10 2015 104 927 A1 ist ein Drehdämpfer zur Dämpfung einer Schwenkbewegung bekannt geworden, der auch bei einer Prothese eingesetzt werden kann. Dabei wird ein magnetorheologisches Medium in einem umlaufenden dünnen Spalt gezielt mit einem Magnetfeld beaufschlagt, um durch Scherkräfte eine Dämpfung einzustellen.

Im Stand der Technik sind verschiedenste Protheseneinrichtungen mit Dämpfern bekannt geworden, mit denen eine Dämpfung einer Schwenkbewegung z. B. eines Knies möglich ist. Gerade bei Knieprotheseneinrichtungen ist das erforderliche oder gewünschte Bremsmoment relativ hoch, um eine sichere und komfortable Funktion zu gewährleisten. Da bei Protheseneinrichtungen der benötigte und zur Verfügung stehende Drehwinkel oder Schwenkwinkel begrenzt ist, sind bekannte Drehdämpfer oftmals nicht flexibel genug anwendbar oder es ist das erforderliche Bremsmoment zu gering, sodass das Bremsmoment gar nicht oder nicht schnell genug verändert oder nicht stark eingestellt werden kann.

Rotationsdämpfer mit Öl und externen Steuerventilen sind Stand der Technik. Besonders bei Protheseneinrichtungen, aber auch bei anderen Anwendungen, ist ein geringer Platzbedarf von großem Vorteil. Dies bedeutet, dass die Wirkflächen klein sind und deswegen der Arbeitsdruck erhöht werden muss (100bar und mehr), damit entsprechende Flächendrücke und damit Kräfte bzw. Momente erzeugt werden können. Nachteilig bei diesen Aktoren ist, dass die sich zueinander bewegenden Teile sehr genau hergestellt werden müssen, damit es in den Spalten zu einem möglichst hohen Druckverlust kommt und somit eine Dichtwirkung erzielt wird. Diese engen Spaltabmessungen und eng tolerierten Gleitpaarungen erhöhen die hydraulische und mechanische Grundreibung/-momente, was sich nachteilig auf die Funktionalität und das Ansprechverhalten auswirkt. In weiterer Folge ist mit hohem mechanischem Verschleiß und kurzen Serviceintervallen zu rechnen. Da es sich hierbei oftmals um Innenkonturen und rechteckige oder unförmige Bauteile/Dichtkanten handelt und diese vorzugsweise geschliffen werden müssen, damit die Toleranzen/Spalte entsprechend gut werden, sind die Kosten hierfür sehr hoch. Das alternative Anbringen von Dichtelementen ist bei diesen Konturen, Drücken ebenso aufwendig und kostenintensiv. Besonders schwierig ist es die Kanten bzw. die Übergänge von z. B. der Axial- zur Radialkontur zu dichten. Zudem verursachen Dichtungen eine hohe Grundreibung bzw. Grundreibungskräfte bzw. -momente.

Es ist deshalb die Aufgabe der vorliegenden Erfindung, eine Protheseneinrichtung mit einem einfach aufgebauten Drehdämpfer zur Verfügung zu stellen, womit eine flexible Einstellung der Dämpfung der ermöglicht, die Dämpfung auch hoher Kräfte und Drehmomente erlaubt wird.

Diese Aufgabe wird durch eine Protheseneinrichtung mit einem Drehdämpfer mit den Merkmalen des Anspruchs 1 gelöst. Bevorzugte Weiterbildungen der Erfindung sind Gegenstand der Unteransprüche. Weitere Vorteile und Merkmale der vorliegenden Erfindung ergeben sich aus der allgemeinen Beschreibung und der Beschreibung der Ausführungsbeispiele.

Eine erfindungsgemäße Protheseneinrichtung umfasst wenigstens einen und ein Gehäuse und eine drehbar daran aufgenommene Dämpferwelle, eine Verdrängereinrichtung in dem Gehäuse und wenigstens eine Magnetfeldquelle. Dabei weist die Verdrängereinrichtung ein Dämpfervolumen mit einem magnetorheologischen Fluid als Arbeitsfluid auf und ist damit betreibbar, um eine Dämpfung der Drehbewegung der Dämpferwelle relativ zu dem Gehäuse zu beeinflussen. Dabei umfasst die Verdrängereinrichtung wenigstens zwei Trenneinheiten, mit denen das Dämpfervolumen bzw. ein Dämpfervolumen in dem Dämpfergehäuse in wenigstens zwei variable Kammern unterteilt wird, wobei wenigstens eine der Trenneinheiten eine mit dem Gehäuse verbundene Trennwand umfasst. Wenigstens eine der Trenneinheiten umfasst eine mit der Dämpferwelle verbundene Trennwand und kann vorzugsweise als Schwenkflügel ausgebildet sein. In radialer Richtung ist zwischen der mit dem Gehäuse verbundenen Trenneinheit und der Dämpferwelle ein (erster) (radialer) Spaltabschnitt oder Spalt ausgebildet. Der erste Spaltabschnitt verläuft im Wesentlichen in axialer Richtung. In radialer Richtung ist zwischen der mit der Dämpferwelle verbundenen Trenneinheit und dem Gehäuse ein weiterer (bzw. ein zweiter) (radialer) Spaltabschnitt ausgebildet. Der weitere oder zweite Spaltabschnitt verläuft wenigstens zu einem erheblichen Teil in axialer Richtung. In axialer Richtung ist zwischen der mit der Dämpferwelle verbundenen Trenneinheit und dem Gehäuse wenigstens noch ein (bzw. ein dritter) (axialer) Spaltabschnitt ausgebildet. Dieser (bzw. der dritte Spaltabschnitt) verläuft wenigstens zu einem erheblichen Teil in radialer Richtung. Wenigstens ein wesentlicher Teil des Magnetfeldes der Magnetfeldquelle durchtritt wenigstens zwei der angeführten Spaltabschnitte.

Jeder Spaltabschnitt kann als separater Spalt ausgebildet sein oder es können zwei oder mehr Spaltabschnitte Teil eines gemeinsamen Spaltes sein.

Jeder Spaltabschnitt hat eine Erstreckungsrichtung bzw. Verlaufsrichtung und eine Spalthöhe quer zu der Verlaufsrichtung. Ein rein axialer Spaltabschnitt verläuft in radialer Richtung und/oder in Umfangsrichtung. Die Spalthöhe erstreckt sich in axialer Richtung. Ein rein radialer Spaltabschnitt erstreckt sich in axialer Richtung und gegebenenfalls auch in Umfangsrichtung.

Hier verlaufen der erste und der zweite Spaltabschnitt besonders bevorzugt im Wesentlichen in axialer Richtung, während sich die Spalthöhe jeweils im Wesentlichen in radialer Richtung erstreckt. Der dritte Spaltabschnitt ist besonders bevorzugt als axialer Spaltabschnitt ausgebildet, sodass sich die Spalthöhe im Wesentlichen in axialer Richtung erstreckt. Der Spaltabschnitt erstreckt sich hingegen im Wesentlichen in radialer Richtung und/oder in Umfangsrichtung.

Die Spalte oder Spaltabschnitte können jeweils linear ausgebildet sein. Jeder Spaltabschnitt kann aber auch eine oder mehrere Krümmungen aufweisen oder nur aus jeweils gekrümmten Spaltbereichen bestehen.

Die erfindungsgemäße Protheseneinrichtung hat viele Vorteile. Ein erheblicher Vorteil der erfindungsgemäßen Protheseneinrichtung besteht darin, dass zwei oder mehr Spaltabschnitte und vorzugsweise alle Spaltabschnitte in dem Drehdämpfer durch das Magnetfeld der Magnetfeldquelle bedarfsweise abgedichtet werden. Dadurch können die Spalte oder Spaltabschnitte mit einer ausreichenden Spalthöhe ausgebildet werden, um eine geringe Grundreibung zur Verfügung zu stellen. Weiterhin wird aber bei aktivem Magnetfeld eine hohe Abdichtung erzielt, sodass hohe Dämpfungswerte ermöglicht werden. Es ist nicht nötig, eine Spalthöhe besonders gering zu wählen, damit keine Leckage auftritt. Eine Leckage wird nicht über das Spaltmaß (Spalthöhe) verhindert, sondern über eine magnetische Abdichtung.

Bei Protheseneinrichtungen mit konventionellen Dichtungen in reinen Ölkreisläufen muss hingegen ein besonders geringes Spaltmaß gewählt werden, wenn eine hohe Abdichtung erreicht werden soll. Dadurch resultieren gleichzeitig auch ein hohes Grundmoment im Leerlauf und ein entsprechend hoher Verschleiß an den Dichtungen. Das wird erfindungsgemäß vermieden.

Vorzugsweise umfasst die Magnetfeldquelle wenigstens eine steuerbare elektrische Spule, um eine Stärke des Magnetfeldes zu beeinflussen. Damit wird eine Stärke der Dämpfung und vorzugsweise auch eine Stärke der Dichtung beeinflusst. Insbesondere durchtritt ein wesentlicher Teil des Magnetfeldes der Magnetfeldquelle wenigstens die beiden Spaltabschnitte und beeinflusst in Abhängigkeit von der Stärke des Magnetfeldes wenigstens die beiden angeführten Spaltabschnitte gleichzeitig.

Über eine einstellbare Stärke des Magnetfeldes kann die Stärke der Dämpfung adaptiv angepasst werden.

Mit einer steuerbaren elektrischen Spule kann ein Magnetfeld gewünschter Stärke flexibel eingestellt werden. Darüber wird eine Dämpfung gewünschter Stärke eingestellt. Gleichzeitig wird dadurch insbesondere auch eine Stärke der Abdichtung wenigstens zweier Spalte und insbesondere aller radialen und axialen Spalte eingestellt. Die Grundreibung ist niedrig, wenn das Magnetfeld gering ist und die Abdichtung ist hoch, wenn der Relativdruck bzw. das Drehmoment hoch ist. Somit kann eine viel höhere Dynamik zur Verfügung gestellt werden als im Stand der Technik, da nicht nur die eigentliche Dämpfung, sondern auch die Dichtung beeinflusst wird.

Tatsächlich wirkt ein Bremsmoment, welches sich additiv aus dem vorhandenen Grundmoment und aus dem Dämpfungsmoment zusammensetzt. Hier werden Grundmoment und Dämpfungsmoment jeweils durch das (zeitlich abhängige und zeitlich steuerbare) wirksame Magnetfeld beeinflusst. Bei kleinen zu dämpfenden Kräften und Momenten wird mit einer kleineren Stärke des Magnetfeldes eine kleinere Grundreibung (Grundmoment) erzeugt. Bei größeren zu dämpfenden Kräften und Momenten wird mit einer größeren Stärke des Magnetfeldes eine größere Grundreibung (Grundmoment) erzeugt. Ein größeres Grundmoment wirkt sich bei einem entsprechend größeren Bremsmoment nicht nachteilig auf das Ansprechverhalten aus. Insbesondere ist ein Verhältnis von Bremsmoment zu einem Grundmoment in einem mittleren Betriebsbereich (insbesondre genau in der Mitte) größer als 2:1 und vorzugsweise größer 5:1 und besonders bevorzugt größer 10:1. In einer besonders bevorzugten Ausgestaltung sind die Spaltabschnitte jeweils als Spalt ausgebildet. Die Spalte können zum Teil ineinander übergehen oder getrennt voneinander ausgebildet sein. Es ist dann möglich, den Begriff Spaltabschnitt in dieser Anmeldung durchgängig durch den Begriff Spalt zu ersetzen.

Unter einer Protheseneinrichtung im Sinne der vorliegenden Erfindung wird nicht nur eine Prothese verstanden, sondern auch eine Exoskeletteinrichtung oder eine Ortheseneinrichtung und insbesondere ein Exoskelett oder eine Orthese.

In einer bevorzugten Ausgestaltung durchtritt ein wesentlicher Teil des Magnetfeldes der Magnetfeldquelle wenigstens einen und insbesondere zwei an gegenüberliegenden Enden ausgebildete axiale Spaltabschnitte zwischen dem Gehäuse und wenigstens einer der Trenneinheiten, um die seitlichen Axialspalte abzudichten. Durch das dort durchtretende Magnetfeld werden die in dem Axialspalt vorhandenen magnetorheologischen Partikel miteinander verkettet, sodass eine vollständige und auch bei hohen Drücken wirksame Abdichtung erfolgt. Alternativ oder zusätzlich kann auch wenigstens ein radialer Spaltabschnitt bzw. Spalt zwischen der mit der Dämpferwelle verbundenen Trenneinheit und dem Gehäuse von dem Magnetfeld beaufschlagt werden, sodass bei aktivem Magnetfeld auch dieser radiale Spalt (Spaltabschnitt) abgedichtet wird.

In einer bevorzugten Weiterbildung sind wenigstens einer der Spaltabschnitte als Dämpfungsspalt und wenigstens einer der Spaltabschnitte als Dichtspalt ausgebildet. Dabei weist wenigstens ein Dämpfungsspalt vorzugsweise eine (erheblich) größere Spalthöhe als ein Dichtspalt auf. Insbesondere ist eine Spalthöhe des Dämpfungsspalts wenigstens doppelt so groß oder wenigstens 4 Mal so groß oder wenigstens 8 Mal so groß wie eine Spalthöhe eines Dichtspalts. Es ist bevorzugt, dass eine Spalthöhe eines Dichtspalts größer 10 µm und insbesondere größer 20 µm und vorzugsweise zwischen etwa 20 µm und 50 µm beträgt. Eine Spalthöhe eines Dämpfungsspalts ist hingegen vorzugsweise >100 µm und vorzugsweise >250 µm und beträgt bevorzugt zwischen 200 µm und 2 mm Spalthöhe. In vorteilhaften Ausgestaltungen kann die Spalthöhe eines Dämpfungsspalts zwischen (etwa) 500 µm und 1 mm betragen.

Grundsätzlich tragen alle Spaltabschnitte zur Dämpfung bei bzw. beeinflussen diese. Ein Durchfluss durch einen Dämpfungsspalt (mit einer größeren Spalthöhe) kann effektiv durch eine Steuereinrichtung gesteuert werden, sodass das wirkende Bremsmoment genau einstellbar ist. Durch einen Dämpfungsspalt mit einer größeren Spalthöhe kann ein entsprechend hoher Volumenstrom transportiert werden.

Vorzugsweise umfasst die Magnetfeldquelle wenigstens eine elektrische Spule. Möglich ist es auch, dass 2, 3 oder mehr elektrische Spulen eingesetzt werden, um das Magnetfeld der Magnetfeldquelle zu bilden. Möglich ist es auch, dass die Magnetfeldquelle wenigstens einen Dauermagneten umfasst oder dass der Magnetfeldquelle wenigstens ein Dauermagnet zugeordnet ist.

In bevorzugten Weiterbildungen ist an beiden axialen Enden der mit der Dämpferwelle verbundenen Trennwand jeweils ein (stirnseitiger) axialer Spaltabschnitt bzw. Spalt zwischen dem Gehäuse und der Trennwand ausgebildet. Vorzugsweise tritt wenigstens ein wesentlicher Teil des Magnetfeldes der Magnetfeldquelle durch beide axialen Spaltabschnitte zwischen dem Gehäuse und der Trennwand und bewirkt eine Dichtung der beiden (stirnseitigen) axialen Spaltabschnitte. Dies Spaltabschnitte sind dann der dritte Spaltabschnitt und ein vierter Spaltabschnitt. Durch das Magnetfeld werden dann die Axialspalte an beiden Stirnseiten abgedichtet. Eine Steuerung des Durchflusses kann auch durch eine Steuerung der Stärke des Magnetfeldes an diesen Dichtspalten beeinflusst werden. Maßgeblich wird der Durchfluss aber durch den oder die Dämpfungsspalte bzw. Dämpfungsspaltabschnitte beeinflusst.

Möglich ist es auch, dass eine nicht rechteckige Trenneinheit eingesetzt wird. Beispielsweise können die Trenneinheiten halbkreisförmig ausgebildet sein und in einer entsprechenden halbkugelförmigen Aufnahme in dem Gehäuse aufgenommen sein. Dann ergeben sich auch Spalte oder Spaltabschnitte mit (teilweiser oder überwiegender) axialer Ausrichtung und mit (teilweiser oder überwiegender) vertikaler Ausrichtung. Unter zwei Spaltabschnitten im Sinne der vorliegenden Erfindung können auch unterschiedliche ausgerichtete Abschnitte eines durchgehenden Spaltes verstanden werden.

Vorzugsweise sind 2 elektrische Spulen vorgesehen, die insbesondere jeweils benachbart zu dem Dämpfervolumen angeordnet sind. Vorzugsweise ist jeweils eine steuerbare elektrische Spule jeweils einem axialen Spalt zugeordnet. Insbesondere ist jeweils eine steuerbare elektrische Spule jeweils axial nach außen in der Nähe eines Axialspaltes untergebracht.

In allen Ausgestaltungen ist es bevorzugt, dass das Magnetfeld quer zu wenigstens einem der Spaltabschnitte verläuft. Insbesondere verläuft das Magnetfeld quer zu zu wenigstens 2, 3 oder mehr Spaltabschnitten. Durch ein sich quer zu dem Spaltabschnitt erstreckendes Magnetfeld wird eine besonders hohe Wirkung erzielt. Dabei kann das Magnetfeld senkrecht zu dem Spaltabschnitt ausgerichtet sein. Das Magnetfeld kann aber auch schräg durch den Spaltabschnitt verlaufen.

Es ist bevorzugt, dass zumindest ein radialer Spaltabschnitt als Dämpfungskanal ausgebildet ist und radial zwischen der mit der Dämpferwelle verbundenen Trenneinheit und dem Gehäuse angeordnet ist. Es ist auch möglich und bevorzugt, dass zumindest ein axialer Spaltabschnitt als Dämpfungskanal ausgebildet ist und axial zwischen der mit der Dämpferwelle verbundenen Trenneinheit und dem Gehäuse angeordnet ist.

Besonders bevorzugt werden sowohl die Axialspalte als auch die Radialspalte durch das Magnetfeld der Magnetfeldquelle abgedichtet werden.

Vorzugsweise tritt wenigstens ein wesentlicher Teil des Magnetfeldes der Magnetfeldquelle durch den Dämpfungskanal durch. Besonders bevorzugt tritt wenigstens ein wesentlicher Teil des Magnetfeldes der Magnetfeldquelle durch alle Spaltabschnitte durch. Unter einem "wesentlichen Teil" des Magnetfeldes wird insbesondere ein Anteil von >10 % und vorzugsweise ein Anteil größer 25 % verstanden.

In allen Ausgestaltungen ist es auch möglich, dass wenigstens ein Spaltabschnitt durch ein mechanisches Dichtmittel abgedichtet ist. Die Aufgabe des Dichtmittels ist es Stoffübergänge und Druckverluste/Druckabfall von einem Raum in einen anderen zu verhindern oder zu begrenzen. Ein solches mechanisches Dichtmittel kann eine mechanische Dichtung wie eine Dichtlippe, Dichtleiste, Flachdichtung, Profildichtung, Einschleifdichtung oder ein O-Ring oder Quadring oder dergleichen sein. Beispielsweise kann der sich zwischen der mit dem Gehäuse verbundenen Trenneinheit und der Dämpferwelle erstreckende Spaltabschnitt durch ein mechanisches Dichtmittel abgedichtet werden, während der Spaltabschnitt zwischen der mit der Dämpferwelle verbundenen Trenneinheit und dem Gehäuse und die axialen Spaltabschnitte mit dem Magnetfeld der Magnetfeldquelle beaufschlagt werden, um die gewünschte Dämpfung einzustellen.

In allen Ausgestaltungen ist es besonders bevorzugt, dass das Gehäuse ein erstes und ein zweites Endteil und dazwischen ein Mittelteil umfasst. Dabei kann insbesondere das Mittelteil auch aus 2 oder mehr separaten Abschnitten bestehen. Insbesondere ist in wenigstens einem der beiden Endteile und insbesondere sind in beiden Endteilen jeweils eine elektrische Spule aufgenommen. Dabei ist eine Achse der Spule insbesondere im Wesentlichen parallel zur Dämpferwelle ausgerichtet. Dadurch wird ein kompakter Aufbau erzielt, bei dem durch das Magnetfeld der Magnetfeldquelle eine hohe Abdichtung erzielbar ist. Ein kompakter Aufbau von Dämpfern von Protheseneinrichtungen ist sehr vorteilhaft.

Vorzugsweise besteht das Gehäuse wenigstens zu einem wesentlichen Teil aus einem magnetisch leitenden Material mit einer relativen Permeabilität größer 100. Insbesondere ist die relative Perversität größer 500 oder größer 1000. Dabei ist es möglich, dass das gesamte Gehäuse aus einem solchen Material besteht oder doch im Wesentlichen oder wenigstens zu einem wesentlichen Teil. Besonders bevorzugt besteht wenigstens einer der an das Dämpfervolumen angrenzenden Gehäuseabschnitte aus einem magnetisch leitenden Material.

Vorzugsweise ist axial benachbart zu der elektrischen Spule in dem Gehäuse ein (separater) Ring angeordnet. Der Ring ist insbesondere axial zwischen der elektrischen Spule und dem Dämpfervolumen angeordnet.

Es ist möglich, dass sich der Ring und/oder die elektrische Spule im Wesentlichen oder nahezu vollständig oder vollständig radial weiter außen befindet als das Dämpfervolumen. Bevorzugt befindet sich der Ring axial benachbart zu und angrenzend an ein Mittelteil des Gehäuses. In solchen Ausgestaltungen ist es bevorzugt, dass der Ring wenigstens im Wesentlichen oder vollständig aus einem Material mit einer relativen Permeabilität kleiner 10 besteht. Die relative Permeabilität des Ringmaterials ist insbesondere kleiner 5 oder sogar kleiner 2. Der Ring besteht insofern vorzugsweise aus magnetisch nicht leitenden Materialien. Der Ring kann zum Beispiel aus austenitischen Stählen bestehen. Das Material des Rings weist eine solche magnetische Permeabilität auf, dass ein magnetischer Kurzschluss des Magnetfeldes der Magnetfeldquelle zuverlässig verhindert wird. Der Ring ist bei derartigen Ausgestaltungen insbesondere als flache Ringscheibe oder als Hohlzylinder ausgebildet.

In anderen Ausgestaltungen ist der Ring und/oder die elektrische Spule (im Wesentlichen) nicht benachbart zu dem Mittelteil des Gehäuses angeordnet. Dann ist es möglich und bevorzugt, dass der Ring und/oder die elektrische Spule radial weiter innen und/oder wenigstens teilweise oder vollständig benachbart zu dem Dämpfervolumen angeordnet sind. Der Ring kann als Hohlzylinder und insbesondere als hohler Kegelstumpf ausgebildet sein. Radial nach außen hin weist der Ring dann eine geringere Wandstärke auf als radial weiter innen. Der Querschnitt durch den Ring weist einen schrägen Verlauf auf. In solchen Ausgestaltungen besteht der Ring vorzugsweise aus einem magnetisch leitenden Material. Die relative Permeabilität des Ringmaterials ist dann vorzugsweise größer 10 und besonders bevorzugt größer 50 und insbesondere größer 100. Die Gestaltung ist sehr vorteilhaft, da dadurch im Bereich der elektrischen Spule eine mögliche Leckage durch den (axialen) Spaltabschnitt zuverlässig verhindert wird. Der Ring weist vorzugsweise die Form eines Kegelstumpfes mit hohlzylindrischem Inneren auf und besteht aus einem magnetisch leitenden Material. Durch eine solche Ausgestaltung wird bei einer Anordnung der Spule seitlich neben dem Dämpfervolumen eine Leckage im Bereich der Spule verhindert, insbesondere, wenn das wirkende Magnetfeld so stark ist, dass das Material des Ringes magnetisch gesättigt ist.

In allen Ausgestaltungen wird durch eine magnetische Abdichtung der Axialspalte an den Stirnseiten die Dämpfung erhöht. Außerdem wird ein Druckverlust innerhalb des Axialspaltes durch überströmendes magnetorheologisches Fluid verhindert.

In allen Ausgestaltungen ist es besonders bevorzugt, dass das magnetorheologische Fluid durch eine relative Schwenkbewegung der Dämpferwelle und des Gehäuses durch wenigstens einen (Dämpfungs-) Spalt von einer Kammer in die andere Kammer gefördert wird.

Es ist möglich und bevorzugt das an der Dämpferwelle 2 oder mehr über dem Umfang verteilt angeordnete Trenneinheiten ausgebildet sind. Dann sind vorzugsweise an dem Gehäuse entsprechend 2 oder mehr über dem Umfang verteilt angeordnete Trenneinheiten ausgebildet. Vorzugsweise wirkt die jeweils eine mit der Dämpferwelle verbundenen Trenneinheit mit einer mit dem Gehäuse verbundenen Trenneinheit zusammen. Durch eine Mehrzahl von Trenneinheiten-Paaren kann das maximal wirksame Bremsmoment erhöht werden.

Wenn nur eine Trenneinheit an der Dämpferwelle ausgebildet ist und nur eine Trenneinheit an dem Gehäuse ausgebildet ist, ist der maximal mögliche Schwenkwinkel zwischen der Dämpferwelle und dem Gehäuse in der Regel kleiner als 360° oder beträgt (fast) 360°. Werden jeweils 2 Trenneinheiten eingesetzt, so beträgt der maximale Schwenkwinkel bis zu (und in der Regel etwas weniger als) 180°. Dementsprechend werden bei 4 Trenneinheiten an der Dämpferwelle und dem Gehäuse regelmäßig nur Schwenkwinkel kleiner 90° bzw. bis zu 90° möglich, was für Protheseneinrichtungen von Kniegelenken in der Regel ausreicht. Wenn hohe Bremsmomente gefordert werden, kann so über einfache Mittel eine Protheseneinrichtung zur Verfügung gestellt werden.

Vorzugsweise werden bei einer entsprechenden Anzahl von Trenneinheiten entsprechend viele Kammern bzw. Kammerpaare gebildet, von denen dann bei einer Schwenkbewegung jeweils ein Teil eine Hochdruckkammer bildet, während ein anderer Teil jeweils eine Niederdruckkammer bildet. Vorzugsweise werden dann die Hochdruck- bzw. Niederdruckkammern durch entsprechende Verbindungskanäle miteinander verbunden, um so jederzeit einen Druckausgleich zwischen den einzelnen Hochdruckkammern bzw. den einzelnen Niederdruckkammern zur Verfügung zu stellen. Die Wirksamkeit des Drehdämpfers insgesamt beeinträchtigen solche Verbindungskanäle nicht, da theoretisch in allen Hochdruckkammern (Niederdruckkammern) jederzeit der gleiche Druck vorherrschen sollte. Es hat sich aber herausgestellt, dass durch entsprechende Verbindungskanäle die Funktionalität verbessert werden kann und allfällige Toleranzen ausgeglichen werden können.

In bevorzugten Ausgestaltungen ist eine Ausgleichseinrichtung mit einem Ausgleichsvolumen vorgesehen. Die Ausgleichseinrichtung dient insbesondere dazu, einen Leckage- und/oder Temperaturausgleich zu ermöglichen. Durch die Ausgleichseinrichtung kann ein Volumenausgleich bei sich ändernden Temperaturen zur Verfügung gestellt werden. Außerdem kann eine verbesserte Langzeitfunktionalität gewährleistet werden, da über ein entsprechendes Ausgleichsvolumen auch über lange Zeit Leckageverluste ausgeglichen werden können, ohne die Funktionalität zu beeinträchtigen.

In bevorzugten Ausgestaltungen aller zuvor beschriebenen Ausführungsformen und Ausgestaltungen ist das Ausgleichsvolumen über eine Ventileinheit mit den beiden Kammern (Hochdruckseite und Niederdruckseite) verbunden. Dabei ist die Ventileinheit vorzugsweise dazu ausgebildet, eine Verbindung zwischen dem Ausgleichsvolumen und einer Niederdruckkammer herzustellen und eine Verbindung zwischen dem Ausgleichsvolumen und der Hochdruckkammer zu blockieren. In einfachen Ausgestaltungen wird diese Funktionalität durch ein Doppelventil einer Ventileinheit zur Verfügung gestellt, wobei beide Ventile der Ventileinheit sich jeweils schließen, wenn in der angrenzenden Kammer ein höherer Druck vorherrscht als in dem Ausgleichsvolumen. Dies führt dazu, dass automatisch Volumen aus dem Ausgleichsvolumen herausgefördert bzw. in das Ausgleichsvolumen hinein gefördert wird, wenn der Druck in der jeweiligen Niederdruckkammer absinkt bzw. ansteigt.

In besonders bevorzugten Ausgestaltungen ist die oder ein Teil der Ausgleichseinrichtung im Inneren der Dämpferwelle untergebracht. Das spart Bauraum. Der ist bei vielen Prothesen knapp. Insbesondere weist die Dämpferwelle einen Hohlraum im Inneren auf. Der Hohlraum ist vorzugsweise von (wenigstens) einem axialen Ende der Dämpferwelle aus zugänglich. Insbesondere ist wenigstens ein Teil des Hohlraums oder der ganze Hohlraum als runder oder regelmäßig ausgebildeter Hohlzylinder ausgebildet. In dem Hohlhohlraum bzw. Hohlzylinder ist vorzugsweise eine Lauffläche für einen Trennkolben ausgebildet, um eine Luft- oder Fluidkammer von einem insbesondere mit MRF gefüllten Ausgleichsvolumen zu trennen. Das Ausgleichsvolumen ist vorzugsweise mit wenigstens einem Verbindungskanal mit wenigstens einer Kammer verbunden, um einen Volumenausgleich bei z. B. Temperaturschwankungen oder bei Leckageverlusten von MRF zur Verfügung zu stellen.

Die Dämpferwelle kann in allen Ausgestaltungen und Weiterbildungen einteilig ausgebildet sein. In Bevorzugten Ausgestaltungen ist die Dämpferwelle zweiteilig oder dreiteilig oder mehrteilig ausgebildet. Vorzugsweise sind die zwei, drei oder mehr Teile drehfest miteinander verbindbar oder koppelbar. In einer Ausgestaltung, bei der in einem hohlen Teil der Dämpferwelle (Hohlwelle) eine Ausgleichseinrichtung aufgenommen ist, wie zuvor beschrieben, ist vorzugsweise eine Anschlusswelle vorgesehen, die mit der Hohlwelle axial verbunden und drehfest gekoppelt ist. Die Anschlusswelle und die Hohlwelle können vorzugsweise axial miteinander verschraubt sein.

In allen Ausgestaltungen ist es bevorzugt, dass von innen zu der Oberfläche des Gehäuses wenigstens ein Kanal führt, der innen mit wenigstens einer Kammer verbunden ist und am äußeren Ende zum Beispiel durch einen Verschluss verschließbar ist. Dann kann bei Bedarf außen eine externe Ausgleichseinrichtung angeschlossen werden. Ein eventuell im inneren der Dämpferwelle vorhandener Hohlraum kann durch einen Einsatz aufgefüllt werden.

Vorzugsweise ist an dem Gehäuse wenigstens ein Sensor und insbesondere wenigstens ein Winkelsensor und/oder wenigstens ein Wegsensor vorgesehen. In bevorzugten Ausgestaltungen können ein absoluter Winkel- oder Wegsensor und/oder ein relativer Winkel-oder Wegsensor vorgesehen sein. Über einen z. B. ungenaueren absoluten Sensor ist dann immer ein ungefährer Wert zur Verfügung, während mit dem relativen Sensor dann bei einer erfolgenden Bewegung ein genauer Wert ermittelt wird, der dann verwendet werden kann. Dadurch liegt beispielsweise nach einem Abschalten immer ein "ungefähr" richtiger Wert vor, mit dem die Steuerung zunächst begonnen werden kann.

An dem Gehäuse und insbesondere an einer Außenseite des Gehäuses ist vorzugsweise wenigstens ein mechanischer Anschlag ausgebildet, der mit der Dämpferwelle zusammenwirkt und eine effektive Drehwinkelbegrenzung zur Verfügung stellt, ohne dass die Trennwände auf Block gehen. Das erleichtert die mechanische Auslegung der Festigkeit der Komponenten.

In allen Ausgestaltungen ist es bevorzugt, dass ein Temperatursensor zur Erfassung einer Temperatur des magnetorheologischen Fluids vorgesehen ist. Mittels eines solchen Temperatursensors kann eine an die aktuell vorherrschende Temperatur angepasste Steuerung durchgeführt werden, sodass sich der Drehdämpfer unabhängig von der Temperatur des magnetorheologischen Fluids immer gleich verhält.

In allen Ausgestaltungen ist es besonders bevorzugt, dass der Dämpfungskreislauf des magnetorheologischen Fluids vollständig innerhalb des Gehäuses angeordnet ist. Dadurch wird ein besonders einfacher und kompakter Aufbau ermöglicht.

Vorzugsweise ist ein Winkelsensor vorgesehen, um ein Maß für eine Winkelposition der Dämpferwelle zu erfassen. Dadurch kann eine winkelabhängige Steuerung der Dämpfung erfolgen. Beispielsweise kann in der Nähe einer Endlage eine erhöhte Dämpfung eingestellt werden.

In allen Ausgestaltungen ist es bevorzugt, dass ein Belastungssensor zur Erfassung eines Kennwerts für ein Drehmoment an der Dämpferwelle vorgesehen ist. Dadurch kann dann eine belastungsabhängige Steuerung erfolgen, um zum Beispiel den noch zur Verfügung stehenden Dämpferweg optimal auszunutzen.

In allen Ausgestaltungen ist es auch bevorzugt, dass wenigstens eine Sensoreinrichtung umfasst ist, welche wenigstens einen Positions- und/oder Abstandssensor zur Erfassung einer Position und/oder eines Abstands von umgebenden Objekten dient. Dabei ist die Steuereinrichtung vorzugsweise dazu ausgebildet und eingerichtet, den Drehdämpfer in Abhängigkeit von den Sensordaten der Sensoreinrichtung zu steuern.

In einer bevorzugten Weiterbildung umfasst die Protheseneinrichtung eine Steuereinrichtung und/oder eine Mehrzahl an insbesondere miteinander verbundener Drehdämpfer.

Insbesondere eine Protheseneinrichtung mit mehreren vernetzten Drehdämpfern ermöglicht vielfältige Anwendungen. So können beispielsweise bei Beinprotheseneinrichtungen mehrere Dämpfer eingesetzt werden, die miteinander vernetzt gesteuert werden.

In allen Ausgestaltungen kann die Protheseneinrichtung mit dem Drehdämpfer vielfältig eingesetzt werden. Ein erheblicher Vorteil der erfindungsgemäßen Protheseneinrichtung besteht darin, dass die Verdrängereinrichtung mit einem magnetorheologischen Fluid als Arbeitsfluid ausgerüstet ist. Dadurch kann von einer Steuereinrichtung gesteuert das Magnetfeld der Magnetfeldquelle in Echtzeit, d. h. in wenigen Millisekunden (kleiner 10 oder 20 ms) eingestellt werden und somit wird in Echtzeit auch das anliegende Bremsmoment an der Dämpferwelle eingestellt.

Der Drehdämpfer der Protheseneinrichtung weist eine Verdrängereinrichtung auf. Die Verdrängereinrichtung weist eine Dämpferwelle und rotierende Verdrängerkomponenten auf. Dabei ist eine Drehbewegung der Dämpferwelle (kontrolliert und gesteuert) dämpfbar. Die Verdrängereinrichtung enthält ein magnetorheologisches Fluid als Arbeitsfluid. Es ist wenigstens eine Steuereinrichtung zugeordnet. Weiterhin ist wenigstens eine Magnetfeldquelle vorgesehen bzw. umfasst, die wenigstens eine elektrische Spule aufweist. Die Magnetfeldquelle ist über die Steuereinrichtung steuerbar und über das Magnetfeld ist das magnetorheologische Fluid beeinflussbar, um eine Dämpfung der Drehbewegung der Dämpferwelle und somit der Anschlusskomponenten der Protheseneinrichtung einzustellen.

Eine solche Protheseneinrichtung sehr vorteilhaft. Ein Vorteil besteht darin, dass die Verdrängereinrichtung mit einem magnetorheologischen Fluid als Arbeitsfluid ausgerüstet ist. Dadurch kann von der Steuereinrichtung gesteuert das Magnetfeld der Magnetfeldquelle in Echtzeit, d. h. in wenigen Millisekunden (kleiner 10 oder 20 ms) eingestellt werden und somit wird in Echtzeit auch das anliegende Bremsmoment an der Dämpferwelle eingestellt, wenn der Drehdämpfer ein entsprechendes Bremsmoment aufgeben soll. Der Aufbau des Drehdämpfers ist einfach und kompakt und benötigt wenig Bauteile, sodass die Protheseneinrichtung kostengünstig herstellbar ist.

Der Aufbau der erfindungsgemäßen Protheseneinrichtung mit einem Drehdämpfer ist einfach und kompakt und benötigt wenig Bauteile, sodass die Protheseneinrichtung selbst als (Groß-) Serienteil kostengünstig herstellbar ist. In allen Ausgestaltungen ist es möglich und bevorzugt, dass die Magnetfeldquelle wenigstens einen (zusätzlichen) Dauermagneten umfasst. Durch einen Dauermagneten kann ein gezieltes statisches Magnetfeld erzeugt werden, um beispielsweise ein Grundmoment bestimmter Höhe zu erzeugen bzw. zur Verfügung zu stellen. Dieses Magnetfeld des Dauermagneten kann durch die elektrische Spule der Magnetfeldquelle gezielt verstärkt oder geschwächt werden, sodass das Magnetfeld vorzugsweise beliebig zwischen 0 und 100 % eingestellt werden kann. Daraus resultiert ein entsprechendes Bremsmoment, welches ebenfalls vorzugsweise zwischen 0 % und 100 % eingestellt werden kann. Bei abgestelltem bzw. auf einen geringen Wert reduziertem Magnetfeld ist es möglich, ein geringes oder sehr geringes Grundmoment der Protheseneinrichtung zu erzeugen.

Möglich und bevorzugt ist es, die Magnetisierung des Dauermagneten durch wenigstens einen magnetischen Impuls einer elektrischen Spule dauerhaft zu verändern. Bei einer solchen Ausgestaltung wird der Dauermagnet durch magnetische Impulse der Spule so beeinflusst, dass die Feldstärke des Dauermagneten dauerhaft verändert wird. Dabei kann die dauerhafte Magnetisierung des Dauermagneten durch den magnetischen Impuls der Magnetfelderzeugungseinrichtung auf einen beliebigen Wert zwischen Null und der Remanenz des Dauermagneten eingestellt werden. Auch die Polarität der Magnetisierung ist veränderbar. Ein magnetischer Impuls zur Einstellung einer Magnetisierung des Dauermagneten ist insbesondere kürzer als 1 Minute und vorzugsweise kürzer als 1 Sekunde und besonders bevorzugt beträgt die Länge des Impulses weniger als 10 Millisekunden.

Als Effekt eines Impulses bleibt die Form und Stärke des Magnetfeldes in dem Dauermagneten dauerhaft erhalten. Die Stärke und Form des Magnetfeldes kann durch zumindest einen magnetischen Puls der Magnetfelderzeugungseinrichtung geändert werden. Durch ein gedämpftes magnetisches Wechselfeld kann eine Entmagnetisierung des Dauermagneten erfolgen.

Als Material für solch einen Dauermagneten mit veränderbarer Magnetisierung eignet sich beispielsweise AlNiCo, es können aber auch andere Materialien mit vergleichbaren magnetischen Eigenschaften eingesetzt werden. Es ist zudem möglich, anstelle eines Dauermagneten den gesamten magnetischen Kreis bzw. Teile von ihm aus einer Stahllegierung mit starkem Restmagnetismus (hoher Remanenz) herzustellen.

Möglich ist es, mit dem Dauermagneten ein dauerhaftes statisches Magnetfeld zu erzeugen, welches durch ein dynamisches Magnetfeld der Spule überlagert werden kann, um die gewünschte Feldstärke einzustellen. Dabei kann durch das Magnetfeld der Spule der aktuelle Wert der Feldstärke beliebig verändert werden. Möglich ist auch der Einsatz zweier getrennt ansteuerbarer Spulen.

In allen Ausgestaltungen ist es bevorzugt, dass der Dauermagnet zumindest teilweise aus einem hartmagnetischen Material besteht, dessen Koerzitivfeldstärke größer als 1kA/m und insbesondere größer als 5kA/m und vorzugsweise größer als 10kA/m ist.

Der Dauermagnet kann zumindest teilweise aus einem Material bestehen, welches eine Koerzitivfeldstärke kleiner als 1000 kA/m und vorzugsweise kleiner als 500 kA/m und besonders bevorzugt kleiner als 100 kA/m aufweist.

In allen Ausgestaltungen ist es bevorzugt, dass wenigstens ein Energiespeicher vorgesehen ist. Insbesondere ist der Energiespeicher wieder aufladbar. Der Energiespeicher ist insbesondere mobil ausgebildet und kann an der Protheseneinrichtung oder an dem Drehdämpfer angeordnet sein oder sogar darin integriert werden. Beispielsweise kann der Energiespeicher als Akkumulator oder Batterie ausgeführt sein.

Der Drehdämpfer kann auch zur Dämpfung einer Drehbewegung zwischen 2 Komponenten dienen, wobei zum Beispiel eine Drehbewegung einer Autotür oder einer Heckklappe eines Kraftfahrzeugs oder einer Flügeltour oder einer Motorhaube gedämpft wird. Möglich ist auch der Einsatz an einer Maschine, um daran Drehbewegungen zu dämpfen.

Eine erfindungsgemäße Protheseneinrichtung kann z. B. für eine Hüfte, einen Fuß oder einen Arm vorgesehen sein und wenigstens ein Drehgelenk mit einem Drehdämpfer umfassen. Möglich ist auch der Einsatz bzw. die Ausbildung der Protheseneinrichtung als Orthese, die wenigstens einen Drehdämpfer umfasst. In entsprechender Weise können auch Orthesen entsprechend ausgerüstet werden.

Der hier beschriebene Drehdämpfer der Protheseneinrichtung kann extrem kompakt bauen und sehr kostengünstig produziert werden. Durch die magnetische Abdichtung über das magnetorheologische Fluid kann eine hohe Dichtwirkung erzielt werden. Es sind hohe Maximaldrücke von 100 bar und mehr erreichbar.

Bei der erfindungsgemäßen Protheseneinrichtung kann der Kraftverlauf stufenlos, variabel und sehr schnell über den an die elektrische Spule angelegten Strom geregelt werden.

Vorteilhaft kann die Protheseneinrichtung auch mit einem Computer oder Smartphone verknüpft werden, um die Protheseneinrichtung einzustellen und/oder dessen Arbeitsweise zu protokollieren. Im Computer oder Smartphone wird dann die ideale Einstellung programmiert.

In die Protheseneinrichtung kann eine Feder integriert sein. Die Feder kann als Torsionsfeder, Spiralfeder, Blattfeder oder Luft-/Gasfeder mit mit anderen Teilen in Wirkverbindung sein.

Bevorzugt sind die beiden Hälften im stromlosen Zustand gekoppelt (z. B. mittels Permanentmagnet oder Remanenz im Magnetfeldkreis) und werden mittels Strom beliebig entkoppelt.

Mittels der erfinderischen Merkmale können selbst bei komplexen Konturen und Konturenübergängen mit wenig technischem Aufwand und Kosten große Druckabfälle erzielt werden.

Eine weitere erfindungsgemäße Protheseneinrichtung in Form von z. B. einer Prothese oder einer Orthese oder eines Exoskeletts umfasst einen Drehdämpfer und ein Gehäuse, wenigstens eine Magnetfeldquelle und ein mit einem magnetorheologischen Fluid ausgerüstetes Dämpfervolumen, welches durch wenigstens eine mit einer Dämpferwelle verbundene Trenneinheit in wenigstens zwei (variable) Kammern unterteilt wird. Zwischen der Trenneinheit und dem Gehäuse sind Spaltabschnitte ausgebildet. Das Gehäuse, die Magnetfeldquelle und die Trenneinheit sind dazu ausgebildet und eingerichtet, dass ein Magnetfeld der Magnetfeldquelle die wesentlichen Spaltabschnitte zwischen der Trenneinheit und dem Gehäuse durchflutet.

Es ist vorzugsweise wenigstens eine Trenneinheit vorgesehen, die mit dem Gehäuse verbunden ist. Insbesondere ist ein Spaltabschnitt zwischen der Trenneinheit und der Welle ausgebildet, der von dem Magnetfeld der Magnetfeldquelle durchflutbar ist.

Insbesondere ist die mit der Welle verbundene Trenneinheit als Schwenkflügel ausgebildet.

Vorteilhafterweise sind zwischen dem Schwenkflügel und dem Gehäuse ein radialer Dämpfungsspalt und zwei axiale Dichtspalte ausgebildet.

Vorzugsweise ist wenigstens eine Magnetfeldquelle mit wenigstens einer steuerbaren elektrischen Spule umfasst. Insbesondere wird in Abhängigkeit von einer Stärke des Magnetfeldes eine Stärke der Dämpfung eingestellt.

Bei einem erfindungsgemäßen Verfahren zum Dämpfen von Bewegungen einer Protheseneinrichtung mit einem Drehdämpfer weist der Drehdämpfer der Protheseneinrichtung wenigstens eine Magnetfeldquelle und ein mit einem magnetorheologischen Fluid ausgerüstetes Dämpfervolumen auf, welches durch wenigstens eine mit einer Dämpferwelle verbundene Trenneinheit in wenigstens zwei Kammern unterteilt wird. Zwischen der Trenneinheit und dem Gehäuse sind Spaltabschnitte ausgebildet. Mit einem Magnetfeld der Magnetfeldquelle werden (bedarfsweise) die wesentlichen Spaltabschnitte zwischen der Trenneinheit und dem Gehäuse durchflutet, um die Dämpfung zu beeinflussen und insbesondere eine Stärke der Dämpfung einzustellen.

Die Magnetfeldquelle umfasst wenigstens eine steuerbare elektrische Spule und steuert über die Stärke des Magnetfeldes eine Stärke der Dämpfung. Das gesteuerte Magnetfeld wirkt vorzugsweise in den wesentlichen Spaltabschnitten gleichzeitig. Damit wird nicht nur die Dämpfung gesteuert, sondern es wird auch die Stärke der Dichtung gesteuert und damit das Grundmoment verändert. Das Grundmoment ist so bei kleinen Magnetfeldstärken erheblich geringer.

Grundsätzlich könnten auch Permanentmagnete zur Spaltabdichtung bei MRF angebracht werden. Dabei können ein Permanentmagnet oder es mehrere Permanentmagnete eingesetzt werden. Grundsätzlich wirken diese dann wie mechanische (Gummi-) Dichtelemente. Das geht auch auf einem verschwenkenden Bauteil und auch innen im Druckbereich. Möglich ist eine solche Abdichtung auch an rechteckigen Flächen. Eine derartige Abdichtung geht mit Elektrospulen (elektrischen Spulen) nicht oder nicht so einfach, da diese in den Magnetkreis praktisch "mittig" integriert werden müssen. Vorzugsweise im drucklosen Bereich und mit fixen Kabeln und rund als Wickelteil. Somit ist die Anbringung viel komplizierter als bei Permanentmagneten. Besonders, wenn man mit möglichst wenig elektrischen Spulen jeweils mehr als einen Spalt oder sogar alle Spalte beeinflussen will. Bei der vorliegenden Erfindung sind die Spulen keinem Druck ausgesetzt und können normal gewickelt werden. In Summe ist die Konstruktion sehr einfach und kostengünstig herstellbar. Außerdem ändert sich das Grundmoment mit der Stärke des erzeugten Magnetfeldes. Bei einem nur sehr geringen oder bei keinem Magnetfeld wird eine sehr geringe Reibung eingestellt, da die Spalte groß sind.

In allen Ausgestaltungen kann man den Schwenkwinkel durch die Anzahl der Trenneinheiten bzw. die Flügelanzahl variieren. Bei einer Trenneinheit wird ein Schwenkwinkel von ca. 300 Grad erreicht. Bei zwei Trenneinheiten beträgt der Schwenkwinkel ca. 120 Grad und bei vier Flügeln ca. 40 Grad. Je mehr Trenneinheiten vorgesehen sin, desto größer ist das übertragbare Moment.

Es ist auch möglich, zwei oder mehrere Trenneinheiten (Schwenkflügel) hintereinander zu schalten, also zu kaskadieren. Eine einzelne Trenneinheit ermöglicht ca. 300 Grad Schwenkwinkel. Wenn man die Ausgangswelle mit dem Gehäuse von einem zweiten Drehdämpfer verbindet, kommt man an der Ausgangswelle vom zweiten Drehdämpfer auf 600 Grad. Bei Anwendungen die mehr als 300 Grad benötigen kann man so den Schwenkwinkel erhöhen. Das kann bei geeigneter Verschachtelung Bauraum sparend verwirklicht werden.

Die Anmelderin behält sich vor, für ein Verfahren zur Herstellung von Protheseneinrichtungen Schutz zu beanspruchen, bei dem zunächst wenigstens ein zwei- oder dreidimensionaler Scan wenigstens eines Teils des Körpers des zukünftigen Benutzers oder einer repräsentativen Nachbildung durchgeführt wird. Mit den dabei gewonnenen Daten kann die grundlegende Form und Größe festgelegt werden, wobei händische Modifizierungen möglich sind. Es kann insbesondere ein Design ausgewählt werden. Vorzugsweise werden die festgelegten Daten der Konstruktion einer Fertigungseinrichtung übergeben, wobei eine solche Fertigungseinrichtung z. B. ein 3D-Drucker sein kann oder wenigstens einen solchen umfassen kann. Nach einer etwaigen Nachbehandlung und/oder Verkleidung des Körpers bzw. Gehäuses der eigentlichen Protheseneinrichtung und der Montage der weiteren Komponenten und Teile (Steuerung, Kabel, Drehdämpferkomponenten, Außenhaut) kann - falls noch nötig - eine mechanische Anpassung an den Körper des Benutzers durchgeführt werden.

Dabei kann die Fertigung auch extern durch z. B. einen Dienstleister wie einen Handwerksbetrieb oder andere Zulieferer erfolgen.

Es besteht im Grundsatz ein z. B. linearer Arbeitsprozess, bei dem heute an jedem Knotenpunkt ein Dienstleister oder ein Industriebetrieb oder ein Service steht. In Zukunft kann der gesamte Prozess durch eine Verschaltung der beteiligten Prozessschritte vereinfacht werden. So können sich einige oder sogar alle zu einem Dienstleister zusammenfügen oder bestimmte Leistungen extern beziehen. Solche externen Dienstleistungen können z. B. durch Hochschulen oder medizinische Einrichtungen, Sporteinrichtungen, durch Militärveteranen oder Rehaeinrichtungen zur angeboten werden. Auch ein Dienstleister mit einem 3D-Drucker kann einbezogen werden.

Bei einzelnen Prozessschritten können auch alternative Möglichkeiten vorgesehen sein. Z. B. beim 3D-Druck könnte ein Alternative ein Unternehmen sein, das die Prothesen liefert, oder der Körper der Prothese wird im CNC gefertigt oder maschinell oder per Hand z. B. aus Holz oder anderen geeigneten Werkstoffen bearbeitet oder z. B. geschnitzt.

Bei dem Prozessschritt Design kann ein Programm oder eine "App" eingesetzt werden, mit dem unter Eingabe (oder Übergabe) spezieller Parameter ein gängiges CAD-Programm bedient (oder ersetzt) wird. Das bedeutet, dass Körperdaten importiert oder eingegeben werden. In z. B. einem Menü kann man das Körpergewicht eingeben oder auswählen. Aus einer bestehenden Bibliothek können bestimmte Designmodelle und/oder technische Parameter ausgewählt werden. Beispielsweise kann auch die Auswahl bestimmter magnetorheologischer Module (MRF-Modul) möglich sein.

Es ist auch möglich, dass ein Programm bzw. das Programm eine geeignete (Körper-) Struktur anbietet.

Ein Service Provider kann auch z. B. eine Schulung anbieten oder eine Installation durchführen.

Optional kann der Kunde bestimmte Parameter über seinen Computer im Allgemeinen oder sein Smartphone im Speziellen Daten eingeben, einen Modus oder Ausstattungsmerkmale auswählen.

Es ist möglich, dass eine Protheseneinrichtung primär nur zum "Dämpfen" vorgesehen ist, insbesondere, wenn keine Feder oder nur eine kleine Feder zum Vorspannen und (schlagartig) Loslassen vorgesehen ist. Möglich ist auch die Integration einer Feder. Dazu kann ein Baukastensystem vorgesehen sein. Möglich ist eine Dämpfung und gegebenenfalls Federung in der Zugstufe und der Druckstufe.

Weitere Vorteile und Merkmale der vorliegenden Erfindung ergeben sich aus der Beschreibung der Ausführungsbeispiele, die im Folgenden mit Bezug auf die beiliegenden Figuren erläutert werden.

In den Figuren zeigen:
- Figur 1: eine erste erfindungsgemäße Protheseneinrichtung mit mehreren Drehdämpfern;
- Figur 2: eine zweite erfindungsgemäße Protheseneinrichtung;
- Figur 3: einen Teilschnitt durch einen Drehdämpfer der Protheseneinrichtung nach Figur 1;
- Figur 4: einen schematischen Schnitt durch einen Drehdämpfer für eine erfindungsgemäße Protheseneinrichtung;
- Figur 5: einen Schnitt durch einen anderen Drehdämpfer für eine erfindungsgemäße Protheseneinrichtung;
- Figur 6: Teilschnitt durch einen Drehdämpfer einer Protheseneinrichtung;
- Figur 7: einen Schnitt durch den Drehdämpfer nach Figur 6;
- Figur 8: einen Schnitt durch einen anderen Drehdämpfer für eine Protheseneinrichtung;
- Figur 9: den Schnitt B - B aus Figur 8;
- Figur 10: ein vergrößertes Detail aus Figur 9;
- Figur 11: einen Querschnitt durch einen Drehdämpfer einer erfindungsgemäßen Protheseneinrichtung mit einem eingezeichneten Magnetfeldverlauf;
- Figur 12: einen anderen Querschnitt durch den Drehdämpfer nach Figur 11 mit eingezeichneten Magnetfeldverlauf;
- Figur 13: einen schematischen Querschnitt durch einen Drehdämpfer einer erfindungsgemäßen Protheseneinrichtung;
- Figur 14: eine Dämpferwelle für eine Protheseneinrichtung in unterschiedlichen Ansichten;
- Figur 15: einen Schnitt durch noch einen Drehdämpfer einer Protheseneinrichtung;
- Figur 16: einen schematischen Querschnitt durch einen weiteren Drehdämpfer einer erfindungsgemäßen Protheseneinrichtung;
- Figur 17: eine Protheseneinrichtung mit einem Drehdämpfer mit einem Torsionsstab;
- Figur 18: einen Teilschnitt durch einen Drehdämpfer einer weiteren erfindungsgemäßen Protheseneinrichtung;
- Figur 19: einen Querschnitt durch den Drehdämpfer der Protheseneinrichtung nach Fig. 18;
- Figur 20: einen Längsschnitt durch den Drehdämpfer der Protheseneinrichtung nach Fig. 18; und
- Figur 21: einen alternative Ausführung des Drehdämpfers der Protheseneinrichtung nach Fig. 18.

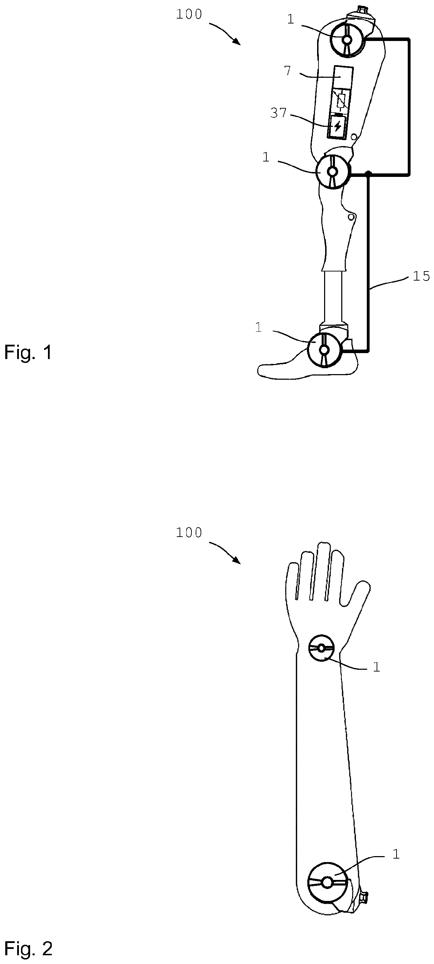

Figur 1 zeigt eine erfindungsgemäße Protheseneinrichtung 100, die hier als Beinprothese 100 ausgeführt ist und die einen oder mehrere Drehdämpfer 1 umfasst. Die erfindungsgemäße Beinprothese 100 umfasst hier drei funktional ähnlich oder gleich ausgebildete Drehdämpfer 1, wobei ein Drehdämpfer 1 am Hüftgelenk, ein weiterer Drehdämpfer 1 am Kniegelenk und ein dritter Drehdämpfer 1 am Fußgelenk vorgesehen sind. Die einzelnen Drehdämpfer 1 sind über Fluidleitungen 15 miteinander verbunden und sind auch elektrisch miteinander gekoppelt, sodass eine vernetzte Steuerung der Protheseneinrichtung 100 möglich ist. Integriert sind in die Protheseneinrichtung 100 auch eine Steuerungseinrichtung 7 und ein Energiespeicher 37. Die Drehdämpfer 1 sind hier auf Schwenkbewegungen bis etwa 180° bzw. bis zu 90° im Falle des Drehdämpfers 1 für das Kniegelenk und für das Fußgelenk ausgelegt.

Figur 2 zeigt den Einsatz an einer Protheseneinrichtung100, die als Unterarmprothese ausgeführt ist, wobei hier ebenfalls zwei miteinander vernetzte Drehdämpfer 1 eingesetzt werden, nämlich einmal am Ellbogengelenk und einmal im Handgelenk.

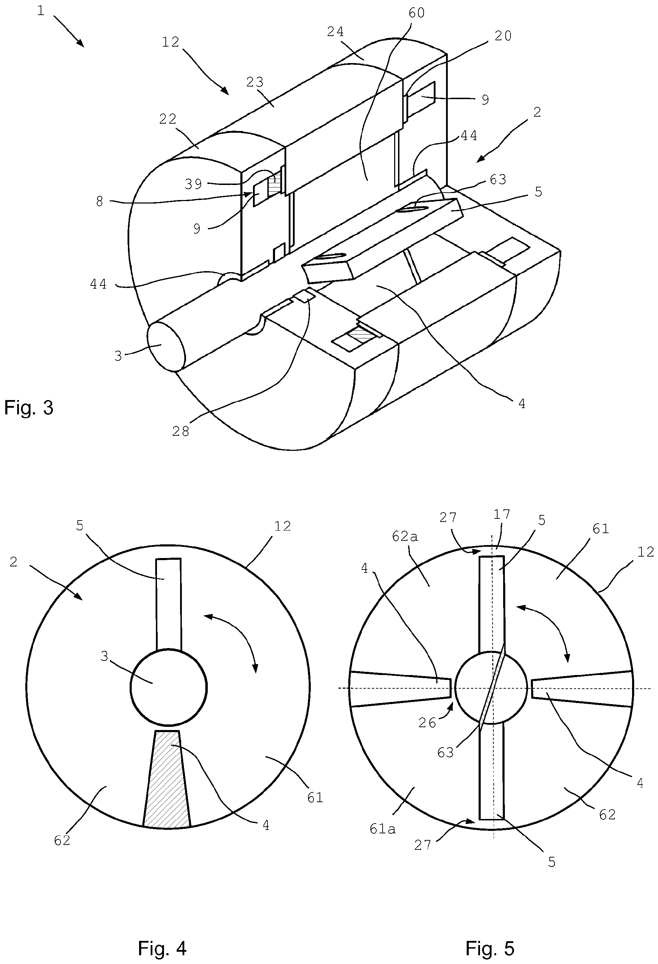

Figur 3 zeigt einen Teilschnitt des Drehdämpfers, der Protheseneinrichtung 100 z. B. aus Figur 1. Der Drehdämpfer 1 der Protheseneinrichtung 100 verfügt über ein Gehäuse 12 und eine Dämpferwelle 3, die gegeneinander verschwenkbar ausgebildet sind. Die Dämpferwelle 3 ist drehbar in dem Gehäuse 12 über Gleitlager 44 gelagert. Das Gehäuse 12 besteht hier aus drei Abschnitten bzw. Gehäuseteilen, nämlich einem ersten Endteil 22 und einem zweiten Endteil 24 am anderen Ende und einem dazwischen angeordneten Mittelteil 23. Hier stellt jeder Teil bzw. jeder Bereich ein separates Bauteil dar, die bei der Montage miteinander verbunden werden. Möglich ist es aber auch, dass die drei Gehäuseteileabschnitte bzw. -bereiche Teil eines einzelnen oder zweier Bauteile bilden.

In den beiden Endteilen 22 und 24 ist jeweils eine umlaufende elektrische Spule 9 aufgenommen, die zur Erzeugung des für die Dämpfung benötigten Magnetfeldes sorgt. Der Innenraum des Drehdämpfers 1 stellt ein Dämpfervolumen 60 zur Verfügung. In dem Gehäuse ist eine Verdrängereinrichtung 2 ausgebildet, die Trenneinheiten 4 und 5 umfasst. Die Trenneinheiten 4 und 5 trennen das Dämpfervolumen 60 in zwei oder mehr Kammern 61 und 62. Dabei ist die Trenneinheit 4 als Trennwand ausgebildet und fest mit dem Gehäuse 12 verbunden. Die Trenneinheit 5 ist ebenfalls als Trennwand oder als Schwenkflügel ausgebildet und ist fest mit der Dämpferwelle 3 verbunden. Vorzugsweise ist die Trenneinheit 5 einstückig mit der Dämpferwelle 3 ausgebildet. Das Dämpfervolumen 60 ist hier mit magnetorheologischen Fluid 6 gefüllt. Eine Abdichtung des Dämpfervolumens 60 nach außen erfolgt über eine Dichtung 28 in dem Gehäuseteil 22. Bei einer Schwenkbewegung verdrängen die Trenneinheiten 4 und 5 das in dem Dämpfervolumen enthaltene magnetorheologische Fluid (MRF), sodass das MRF zum Teil von der einen Kammer in die andere überströmt.

Die Magnetfeldquelle 8 in dem Gehäuseteil 22 besteht hier aus elektrischen Spulen 9 und kann weiterhin wenigstens einen Dauermagneten 39 umfassen, die jeweils ringförmig ausgebildet sind und in dem Gehäuseteil 22 aufgenommen sind. Hier im Ausführungsbeispiel sind in beiden Endteilen elektrische Spulen 9 und gegebenenfalls auch Dauermagneten 39 vorgesehen. Der Dauermagnet 39 gibt eine bestimmte Magnetfeldstärke vor, die über die elektrische Spule 9 moduliert werden kann und so aufgehoben oder verstärkt werden kann.

In das Dämpfervolumen 60 ragen hier zwei Trenneinheiten 4 von dem Gehäuse radial nach innen. Die Trenneinheiten 4 bilden Trennwände und begrenzen so die mögliche Drehbewegung der Dämpferwelle 3, an der ebenfalls 2 Trenneinheiten 5 ausgebildet sind, die von der Dämpferwelle radial nach außen ragen. Durch Drehen der Dämpferwelle 3 werden die Trennwände 5 verschwenkt, die hier Schwenkflügel bilden.

Die elektrischen Spulen 9 sind hier im Ausführungsbeispiel radial relativ weit außen angeordnet und werden hier axial nach innen jeweils von einem magnetisch nicht oder nur schlecht leitenden Ring 20 begrenzt, der zu Formung des Magnetfeldverlaufes dient. Der Ring 20 weist eine hohlzylindrische Form auf.

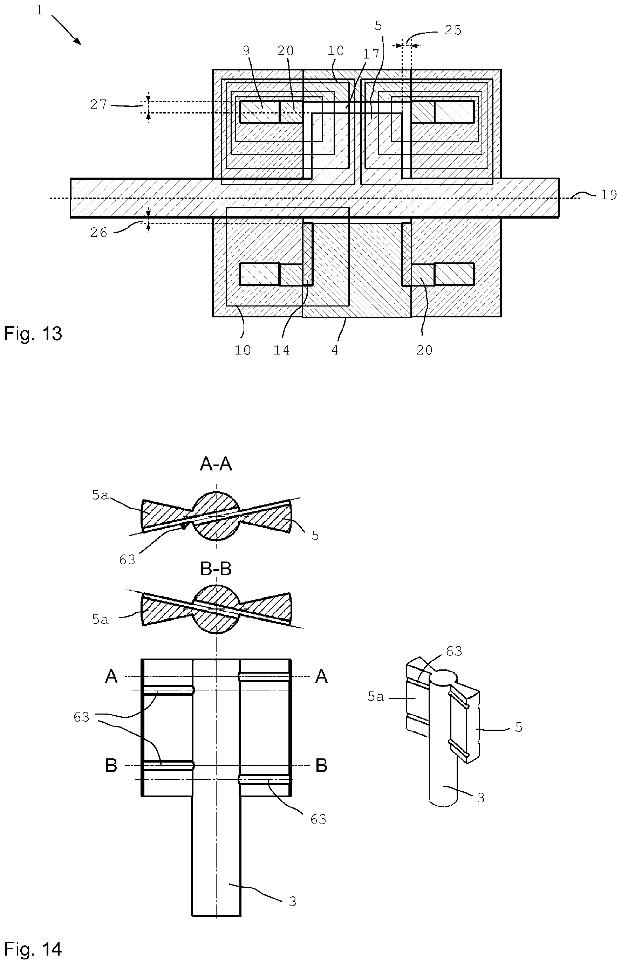

In den Trenneinheiten 5 sind hier Verbindungskanäle 63 erkennbar, die bei der Erläuterung von Figur 5 und 14 noch näher beschrieben werden.

Figur 4 zeigt einen Querschnitt durch einen einfach aufgebauten Drehdämpfer 1 einer Protheseneinrichtung 100. Die Verdrängereinrichtung umfasst hier nur eine (einzige) Trenneinheit 4, die sich radial nach innen von dem Gehäuse aus in das Dämpfervolumen 60 hinein erstreckt. Im Inneren des Gehäuses ist die Dämpferwelle 3 drehbar aufgenommen, an der sich hier auch nur eine Trenneinheit 5 radial nach außen erstreckt. Durch die als Trennwände dienenden Trenneinheiten 4 und 5 der Verdrängereinrichtung 2 wird das Dämpfervolumen 60 in zwei Kammern 61 und 62 variabel aufgeteilt. Bei einer Drehung der Dämpferwelle in Uhrzeigerrichtung wird das Volumen der Kammer 61 verkleinert und das Volumen der Kammer 62 vergrößert, während bei einer umgekehrten Drehbewegung sich das Volumen der Kammer 61 entsprechend vergrößert.

Figur 5 zeigt einen Querschnitt durch ein anderes Ausführungsbeispiel, wobei hier jeweils zwei Trenneinheiten an dem Gehäuse und der Dämpferwelle 3 befestigt sind. Die jeweils symmetrisch angeordneten Trenneinheiten 4 und 5 ermöglichen so eine Schwenkbewegung der Dämpferwelle 3 um nahezu 180°. Zwischen den einzelnen Trenneinheiten 4 und 5 werden zwei Kammern 61 und 61a und 62 und 62a gebildet. Wird die Dämpferwelle 3 im Uhrzeigersinn gedreht, bilden die Kammern 61 und 61a die Hochdruckkammern, während die Kammern 62 und 62a dann Niederdruckkammern sind.

Um einen Druckausgleich zwischen den beiden Hochdruckkammern 61 und 61a zu bewirken, sind entsprechende Verbindungskanäle 63 zwischen den Kammern 61 und 61a und 62 und 62a vorgesehen.

Zwischen dem radial äußeren Ende der Trenneinheiten 5 und dem Innenumfang des prinzipiell zylinderförmigen Dämpfervolumens 60 ist ein radialer Spalt 27 ausgebildet, der hier als Dämpfungskanal 17 dient. Des weiteren sind radiale Spalte 26 zwischen dem radial inneren Ende der Trenneinheiten 4 und der Dämpferwelle 3 ausgebildet. Die Spalte 26 sind dabei so bemessen, dass eine einwandfreie Drehbarkeit der Dämpferwelle 3 ermöglicht wird und dass ein Verklemmen der magnetorheologischen Partikel in dem magnetorheologischen Fluid innerhalb des Dämpfervolumens 60 an den Spalten 26 zuverlässig vermieden wird. Dazu muss der Spalt 26 wenigstens eine größere Spalthöhe aufweisen als der größte Durchmesser der Partikel in dem magnetorheologischen Fluid.

Ein derartig großer Spalt 26 in der Größe von etwa 10 µm bis 30 µm würde normalerweise dafür sorgen, dass ein erheblicher Leckagestrom durch den Spalt 26 fließt. Dadurch würde ein hoher Druckaufbau in den Kammern 61 bzw. 62 effektiv verhindert. Das wird erfindungsgemäß dadurch verhindert, dass der Spalt 26 ebenfalls mit einem magnetischen Feld beaufschlagt wird, sodass auch eine magnetorheologische Abdichtung des Spaltes 26 erfolgt, wenigstens, wenn ein Bremsmoment angelegt werden soll. Dadurch erfolgt eine zuverlässige Abdichtung, sodass ein Druckverlust weitestgehend vermieden werden kann.

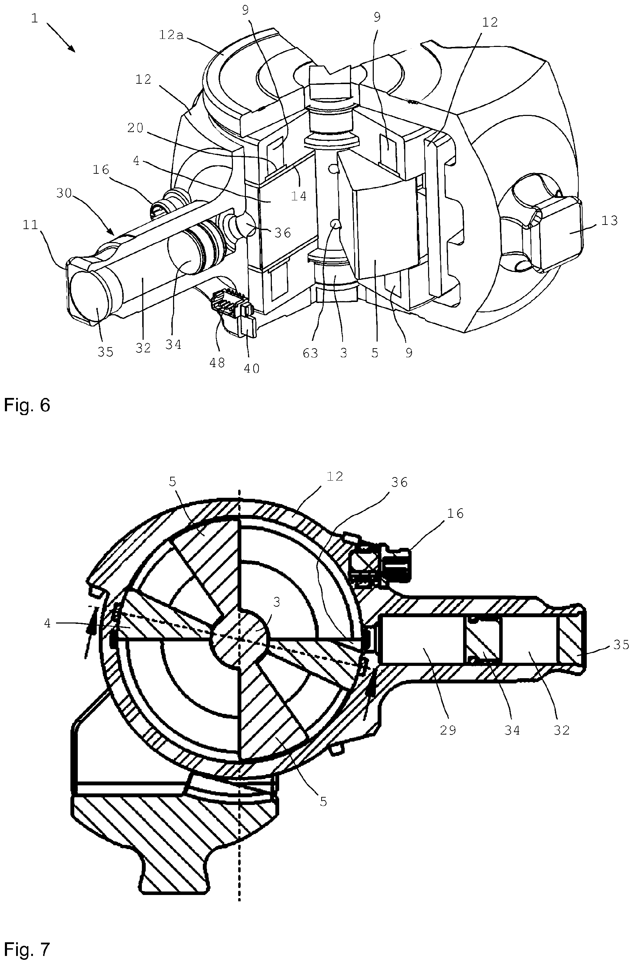

Figur 6 zeigt ein anderes Ausführungsbeispiel einer erfindungsgemäßen Protheseneinrichtung mit einem Drehdämpfer 1. Der Drehdämpfer 1 verfügt über eine Dämpferwelle 3, die in einem Gehäuse 12 drehbar gelagert ist. Die Dämpferwelle 3 bzw. das Gehäuse sind mit relativ zueinander verschwenkbaren Anschlüssen 11 und 13 verbunden.

Das Dämpfervolumen 60 wird durch Trenneinheiten 4 und 5 wieder in Kammern 61 und 62 aufgeteilt, so wie es bei dem Ausführungsbeispiel gemäß Figur 5 der Fall ist.

Auch hier besteht das Gehäuse 12 aus 3 Gehäuseabschnitten oder Gehäuseteilen, wobei in den axial äußeren Gehäuseteilen jeweils eine elektrische Spule 9 zur Erzeugung des benötigten Magnetfeldes aufgenommen ist.

Über einen Stromanschluss 16 wird der Drehdämpfer 1 mit elektrischer Energie versorgt. Eine Sensoreinrichtung 40 dient zur Erfassung der Winkelposition. Außerdem ist es möglich, ein Maß für eine Temperatur des magnetorheologischen Fluids mit der Sensoreinrichtung zu erfassen. Die Weiterleitung der Signale erfolgt über die Sensorleitung 48.

Die Trenneinheit 4 ist ortsfest in dem Gehäuse 12 aufgenommen und wird vorzugsweise bei der Montage in das Gehäuse eingesetzt und damit fest verbunden. Um einen magnetischen Kurzschluss in den Bereichen der Trenneinheit 4 zu verhindern, wird vorzugsweise ein Isolator 14 zwischen der Trenneinheit 4 und den Gehäuseteilen 22 bzw. 24 vorgesehen.

In Figur 6 ist die Ausgleichseinrichtung 30 zu sehen, die eine Luftkammer 32 umfasst, die nach außen durch einen Deckel 35 abgeschlossen wird. Nach innen hin schließt sich an die Luftkammer 32 der Trennkolben 34 an, der die Luftkammer 32 von dem Ausgleichsvolumen 29 trennt. Das Ausgleichsvolumen 29 ist mit magnetorheologischen Fluid gefüllt und stellt einen Ausgleich bei Temperaturschwankungen zur Verfügung. Außerdem dient das Ausgleichsvolumen 29 als Reservoir für Leckageverluste, die sich im laufenden Betrieb ergeben.

Figur 7 zeigt einen Querschnitt durch den Drehdämpfer der Protheseneinrichtung nach Figur 6, wobei hier erkennbar ist, dass jeweils 2 sich gegenüberliegende Trenneinheiten 4 bzw. 5 in dem Gehäuse angeordnet sind bzw. an der Dämpferwelle 3 befestigt sind. Zwischen den einzelnen Trenneinheiten 4 und 5 ergeben sich in dem Dämpfervolumen 60 Kammern 61 bzw. 61a und 62 bzw. 62a. Dadurch, dass jeweils zwei Trenneinheiten 4 bzw. 5 eingesetzt werden, kann das einwirkende Drehmoment verdoppelt werden. Das Ausgleichsvolumen 29 ist über einen Kanal 36 angeschlossen.

Der Kanal 36 wird am Rand der Trenneinheit 4 in das Dämpfervolumen 60 geführt, damit auch bei maximaler Schwenkbewegung zwischen der Dämpferwelle 3 und dem Gehäuse 12 eine Verbindung mit dem Ausgleichsvolumen 29 zur Verfügung steht. In dieser Ausgestaltung muss das Ausgleichsvolumen unter den maximalen Betriebsdruck vorgespannt werden, indem die Luftkammer 32 mit einem entsprechenden Druck beaufschlagt wird. Die Vorspannung kann auch durch ein mechanisches Element wie eine Spiralfeder aufgebracht werden.

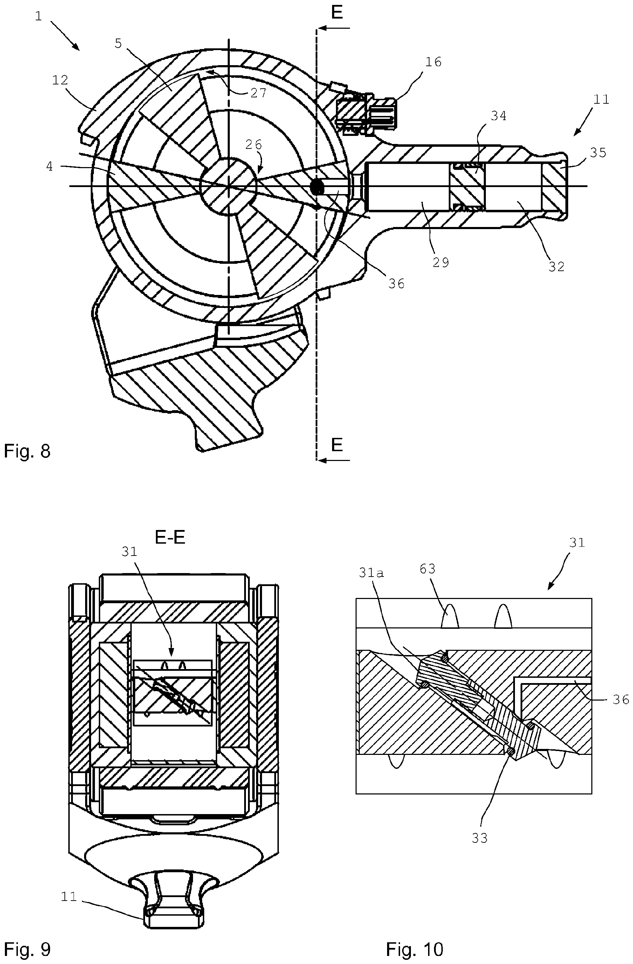

Figur 8 zeigt einen Querschnitt durch einen Drehdämpfer 1 eines weiteren Ausführungsbeispiels einer erfindungsgemäßen Protheseneinrichtung 100. Der Drehdämpfer 1 verfügt wiederum über jeweils zwei Trenneinheiten 4 und 5, die jeweils mit dem Gehäuse bzw. der Dämpferwelle 3 verbunden sind. Auch hier sind zwei elektrische Spulen vorgesehen, die in der Darstellung gemäß Figur 8 aber nicht sichtbar sind, da sie einmal vor und einmal hinter der Schnittebene angeordnet sind.

Radial außen ist zwischen der inneren Gehäusewand und dem radial äußeren Ende der Trennelemente 5 ein Spalt 27 ausgebildet, der zur Dämpfung mit einem entsprechenden Magnetfeld beaufschlagt wird. Radial innen ist zwischen dem inneren Ende der Trennelemente 4 und der Dämpferwelle 3 jeweils ein Spalt 26 ausgebildet, der über ein Magnetfeld abgedichtet wird.

Im Unterschied zum vorhergehenden Ausführungsbeispiel ist hier das Ausgleichsvolumen zentrisch angeschlossen. Das Ausgleichsvolumen 29 wird über den Kanal 36 mit dem Inneren einer Trenneinheit 4 verbunden.

Figur 9 zeigt den Querschnitt B - B aus Figur 8 und Figur 10 zeigt ein vergrößertes Detail aus Figur 10. Der Kanal 36 ist schematisch in Figur 10 eingezeichnet und ist mit einem Kanal verbunden, in dem eine Ventileinheit 31 angeordnet ist, die hier als Doppelventileinheit ausgebildet ist. Die Ventileinheit 31 umfasst zwei Ventilköpfe 31a an den gegenüberliegenden Enden des Kanals. Dichtungen 33 dienen zur Abdichtung, wenn der jeweilige Ventilkopf 31 in seinem Ventilsitz angeordnet ist. Der Kanal 36 mündet in einem Zwischenbereich.

Auf der Seite, auf der der größere Druck vorherrscht, wird der Ventilkopf 31 der Ventileinheit 31 in den entsprechenden Ventilsitz gedrückt. Auf der andern Seite hebt dadurch der Ventilkopf 31a von dem Ventilsitz ab und ermöglicht eine freie Strömungsverbindung zu dem Kanal 36 und somit zu dem Ausgleichsvolumen 29. Dadurch können Temperaturschwankungen ausgeglichen werden. Außerdem wird beim Auftreten von Leckageverlusten magnetorheologisches Fluid aus dem Ausgleichsvolumen in das Dämpfervolumen überführt.

Ein Vorteil dieser Konstruktion ist, dass das Ausgleichsvolumen nur unter einem relativ geringen Vorspannungsdruck von 2, 3 oder 4 oder 5 bar vorgespannt sein muss, da das Ausgleichsvolumen immer mit der Niederdruckseite und nicht mit der Hochdruckseite des Drehdämpfers verbunden ist. Eine solche Ausgestaltung verringert die Belastung auf die Dichtungen und erhöht die Langzeitstabilität. Wird das Ausgleichsvolumen mit der Hochdruckseite verbunden, kann ein Vorspanndruck von 100 und mehr bar sinnvoll sein.

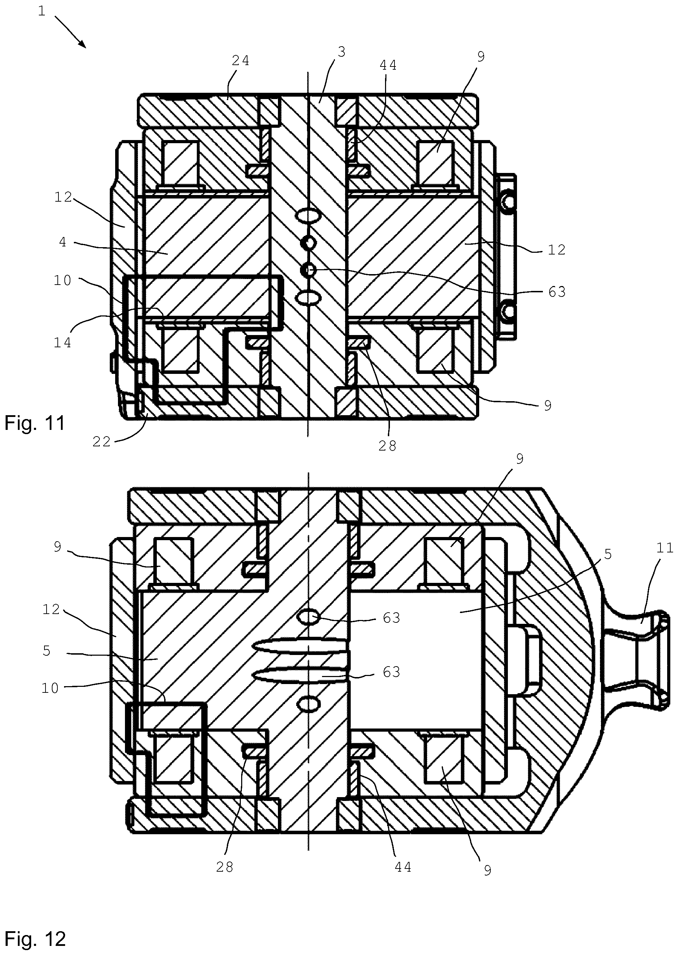

Figur 11 und 12 zeigen Querschnitte durch den Drehdämpfer 1 der Protheseneinrichtung 100, wobei unterschiedliche Querschnitte dargestellt sind. Figur 11 zeigt einen Querschnitt, wobei die mit dem Gehäuse verbundenen Trenneinheiten 4 im Schnitt dargestellt sind. Durch den magnetischen Isolator zwischen den Gehäuseseitenteilen 22 und 24 und der Trennwand 4 bedingt ergibt sich der eingezeichnete Verlauf der Magnetfeldlinie. Dabei durchtreten die Magnetfeldlinien den radial inneren Spalt 26 zwischen dem inneren Ende der Trenneinheiten 4 und der Dämpferwelle 3 und dichten somit dort den Spalt zuverlässig ab. Wenn das Magnetfeld ausgeschaltet wird, wird auch die Dämpfung reduziert und es ergibt sich eine niedrige Grundreibung.

Im Schnitt gemäß Figur 11 sind auch die Gleitlager 44 zur Lagerung der Schwenkwelle und die Dichtungen 28 zur Abdichtung des Innenraums zu erkennen.

Figur 12 zeigt einen Querschnitt durch einen Drehdämpfer 1 einer Protheseneinrichtung, wobei hier der Schnitt durch die Dämpferwelle 3 und eine damit verbundene Trenneinheit 5 verläuft. Die andere mit der Dämpferwelle 3 verbundene und gegenüberliegende Trenneinheit 5 ist hier nicht geschnitten dargestellt. Auch in Figur 12 ist beispielhaft der Verlauf einer Magnetfeldlinie eingezeichnet. Hier wird deutlich, dass die Axialspalte 25 zwischen der Trenneinheit 5 und den Gehäuseteilen 22 und 24 durch das Magnetfeld abgedichtet werden. Des weiteren wird auch der radiale Spalt 27 zwischen einem radial äußeren Ende der Trenneinheit 5 und dem Gehäuse mit dem Magnetfeld beaufschlagt, sodass dort die magnetorheologischen Partikel verketten und den Spalt abdichten.

Figur 13 zeigt nochmals einen schematischen und nicht maßstäblichen Querschnitt durch eine Dämpfereinrichtung 1, wobei hier in der oberen Hälfte ein Schnitt durch die Dämpferwelle 3 und die damit verbundene Trenneinheit 5 dargestellt ist, während in der unteren Hälfte ein Schnitt durch die mit dem Gehäuse verbundene Trenneinheit 4 eingezeichnet ist. Beispielhaft sind jeweils Magnetfeldlinien eingezeichnet. Zwischen der Trenneinheit 4 und der Dämpferwelle besteht ein dünner Spalt 26, der vorzugsweise einer Spalthöhe zwischen etwa 10 und 50 µm aufweist. In axialer Richtung liegt die Trenneinheit 4 dicht an den seitlichen Gehäuseteilen an. Zwischen der Trenneinheit 5 und dem Gehäuse 12 besteht ein radialer Spalt 27 und an den beiden axialen Stirnseiten jeweils ein axialer Spalt 25.

In der Regel sind die Axialspalte 25 mit einer erheblich geringeren Spalthöhe versehen als der radiale Spalt 27. Die Spaltweite der axialen Spalte 25 ist vorzugsweise ähnlich wie die Spaltweite der radialen Spalte 26 und beträgt vorzugsweise zwischen etwa 10 und 30 µm. Die radiale Spaltweite 27 ist vorzugsweise erheblich größer und liegt vorzugsweise zwischen etwa 200 µm und 2 mm und besonders bevorzugt zwischen etwa 500 µm und 1 mm.

Bei der Verschwenkung der Dämpferwelle 3 wird das Volumen einer Kammer verkleinert und dass der anderen Kammer vergrößert. Dabei muss das magnetorheologische Fluid im Wesentlichen durch den Spalt 27 von der einen in die andere Kammer übertreten. Der Spalt 27 dient hier als Dämpfungskanal 17. Wie in Figur 13 anschaulich zu erkennen, wird der Dämpfungskanal 17 von den Magnetfeldlinien durchtreten, sodass dort ein variabler Strömungswiderstand erzeugt werden kann.

Auch die Axialspalte 25 werden durch das Magnetfeld abgedichtet, jedenfalls dann, wenn dessen Magnetfeld so stark gewählt wird, dass es nicht mehr allein durch die Dämpferwelle 3 geleitet wird. Es hat sich nämlich herausgestellt, dass mit stärker werdendem Magnetfeld das gesamte Magnetfeld nicht mehr durch die Dämpferwelle 3 geleitet wird, sondern auch axial den Axialspalt 25 durchtritt und somit mit zunehmender Stärke den vollständigen Axialspalt 25 abgedichtet. Mit einer entsprechenden Feldstärke wird entsprechend abgedichtet.

Wie schon zuvor beschrieben, dienen in diesem Fall die hier magnetisch nicht leitenden Ringe 20 dazu, einen magnetischen Kurzschluss an der elektrischen Spule 9 zu verhindern.

Figur 14 zeigt verschiedene Ansichten der mit zwei Trenneinheiten ausgerüsteten Dämpferwellen 3, wobei sich die Trenneinheiten 5 und 5a diagonal gegenüberliegen, sodass sich ein symmetrischer Aufbau ergibt. In Figur 14 sind die zwei Verbindungskanäle 63 zu erkennen, die jeweils 2 gegenüberliegende Kammern 61 und 61a bzw. 62 und 62a miteinander verbinden. Um einen Druckausgleich zwischen den beiden Hochdruckkammern und den beiden Niederdruckkammern zu ermöglichen, während ein Druckaustausch bzw. ein Fluidaustausch von einer Hochdruckkammer und einer Niederdruckkammer nur über den Dämpfungskanal 17 möglich ist.

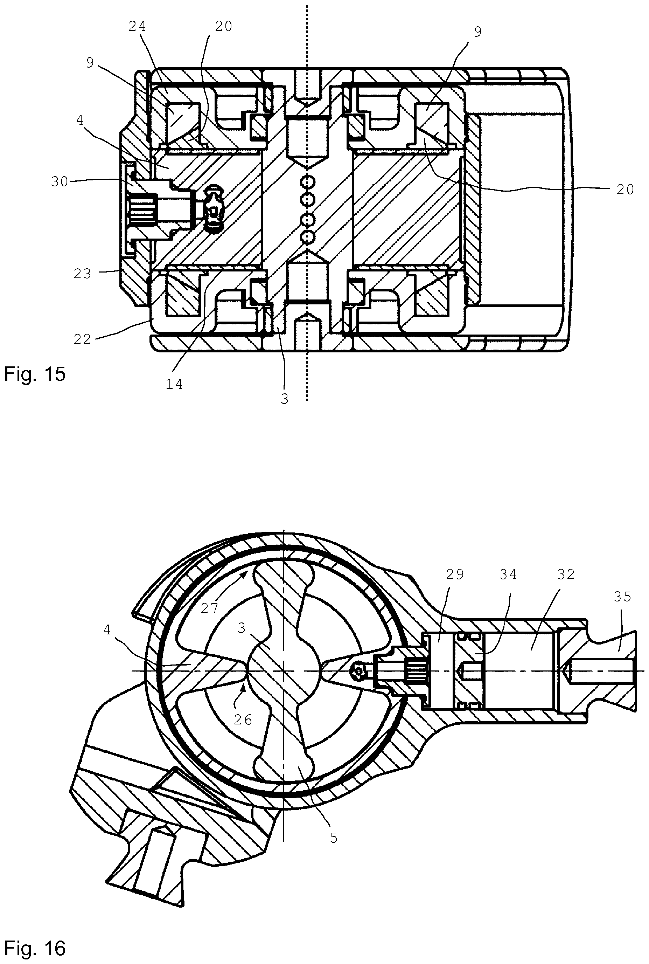

Figur 15 zeigt einen Querschnitt durch einen Drehdämpfer 1 einer weiteren Protheseneinrichtung. Dieser Drehdämpfer baut besonders klein, sodass eine klein bauende und leichte Protheseneinrichtung ermöglicht wird. Der Drehdämpfer 1 aus Figur 15 kann in allen Ausführungsbeispielen eingesetzt werden und ist vom Aufbau grundsätzlich gleich. Im Schnitt zu sehen sind die mit dem Gehäuse verbundenen Trenneinheiten 4. Durch den magnetischen Isolator 14 zwischen den Gehäuseseitenteilen 22 und 24 und der Trennwand 4 bedingt ergibt ein Verlauf der Magnetfeldlinien ähnlich zur Figur 11. Wenn das Magnetfeld ausgeschaltet wird, wird auch hier die Dämpfung reduziert und es ergibt sich eine niedrige Grundreibung. Der Ring 20 ist hier magnetisch leitend ausgebildet, um im Bereich des Trennelementes 5 eine sichere Abdichtung der seitlichen Axialspalte 26 zu gewährleisten. Die Abdichtung wird sicher erreicht, wenn eine ausreichende magnetische Feldstärke vorliegt.. Auch hier sind wie in Figur 11 die Gleitlager 44 zur Lagerung der Schwenkwelle und die Dichtungen 28 zur Abdichtung des Innenraums zu erkennen.

Die elektrischen Spulen 9 sind radial in dem Bereich des Dämpfervolumens angeordnet. Im Bereich der Schwenkflügel wird durch die mit einem Hohlzylinder versehene Kegelstumpfform der Ringe 20 eine sichere Abdichtung auch der seitlichen Axialspalte 26 erreicht. Die hier aus magnetisch leitenden Material bestehenden Ringe 20 sorgen für eine zuverlässige Abdichtung der axialen Dichtspalte 26 im Bereich der Schwenkflügel bzw. Trennelemente 5.

Figur 16 zeigt eine Variante ähnlich Figur 7, wobei hier jeweils wieder zwei Trenneinheiten an dem Gehäuse und der Dämpferwelle 3 befestigt sind. Die jeweils symmetrisch angeordneten Trenneinheiten 4 und 5 ermöglichen so eine Schwenkbewegung der Dämpferwelle 3 um fast 180°. Zwischen den einzelnen Trenneinheiten 4 und 5 werden jeweils zwei Hochdruckkammern und zwei Niederdruckkammern gebildet. Die Trenneinheiten 4 und 5 sind hier abgerundet und strömungsgünstig ausgebildet, damit kein Strömungsabriss erfolgt und damit unerwünschten Ablagerungen aus dem magnetorheologischen Fluid vermieden werden. Es ist auch eine Ausgleichseinrichtung 30 mit einem Ausgleichsvolumen 29 vorgesehen.

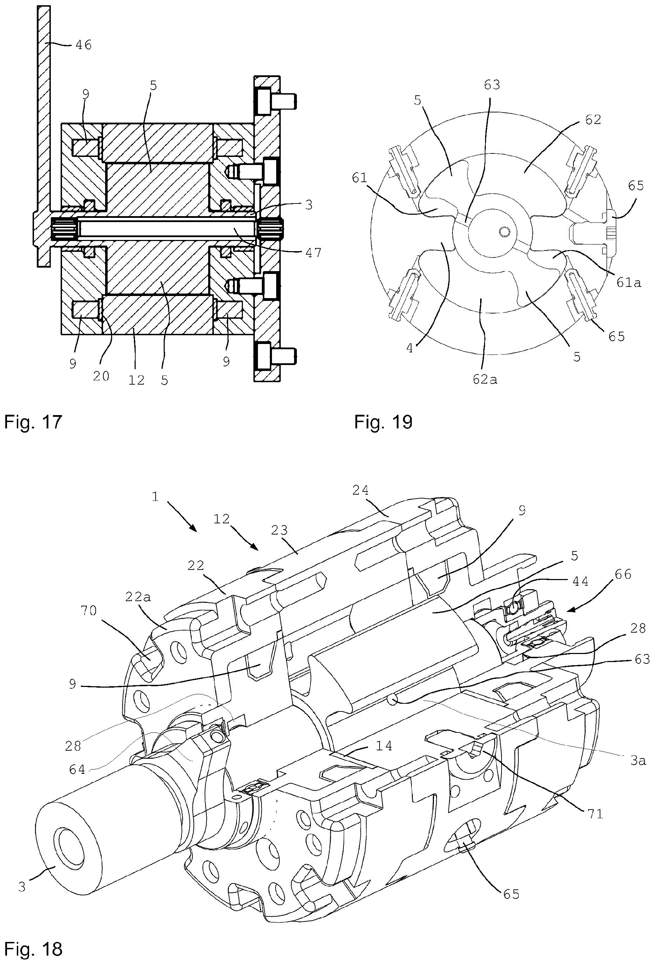

Figur 17 zeigt schließlich noch ein Ausführungsbeispiel, wobei hier der Drehdämpfer 1 der Protheseneinrichtung 100 zusätzlich mit einer Feder in Form eines Torsionsstabes ausgerüstet ist. Es wird die Dämpferwelle mit einer Seite und das Gehäuse mit der anderen Seite gekoppelt, sodass eine Relativbewegung bzw. Relativdrehung der Komponenten zueinander über den Drehdämpfer 1 gesteuert dämpfbar ist. Die Komponenten können einstellbar und auch komplett entkoppelbar sein. Dadurch wird eine aktive Protheseneinrichtung zur Verfügung gestellt, die für unterschiedliche Bedingungen eingestellt werden kann.

In Figur 17 ist weiterhin die Dämpferwelle 3 hohl ausgeführt. Im Inneren der Dämpferwelle ist die Feder in Form von zum Beispiel einem Torsionsstab angeordnet, sodass eine Rückstellung durch die Federkraft der Feder 47 möglich ist.

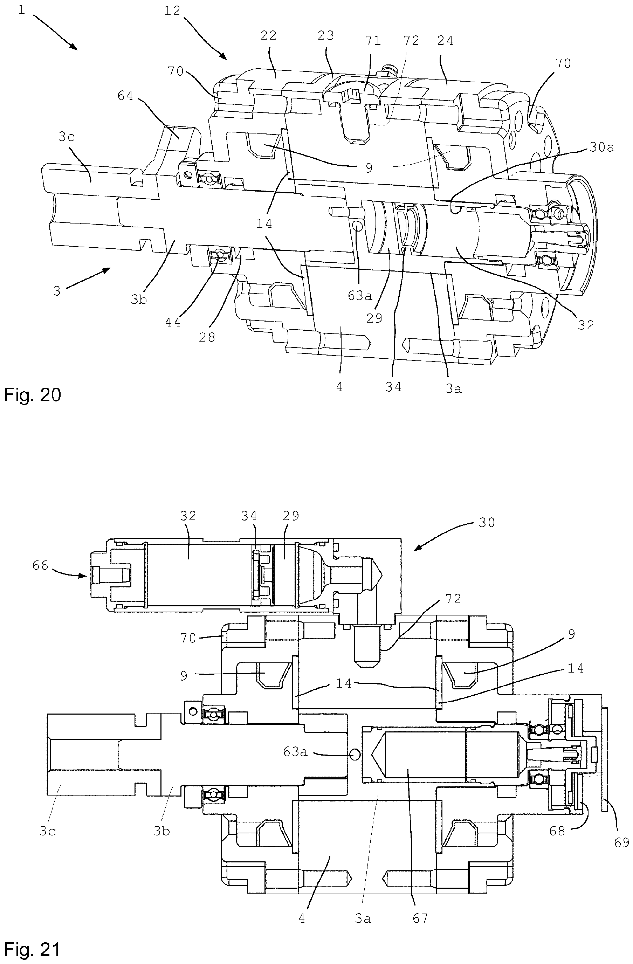

Figur 18 zeigt einen weiteren Drehdämpfer 1 einer weiteren Protheseneinrichtung 100 in einem Teilschnitt, wobei der Drehdämpfer 1 grundsätzlich genauso funktioniert wie z. B. der Drehdämpfer der Protheseneinrichtung 100 nach Fig. 3. Deshalb werden soweit möglich auch die gleichen Bezugszeichen verwendet und die obige Beschreibung gilt identisch auch für die Protheseneinrichtung 100 bzw. den zugehörigen Drehdämpfer 1 der Figuren 18-20, sofern hier keine gegenteilige oder ergänzende Beschreibung erfolgt oder sich aus den Zeichnungen entsprechendes ergibt. Fig. 21 zeigt eine Variante des Drehdämpfers 1 der Protheseneinrichtung 100 nach Fig. 18.

Der Drehdämpfer 1 für eine Protheseneinrichtung 100 oder ein Exoskelett aus Fig. 18 verfügt ebenfalls über ein Gehäuse 12 und eine Dämpferwelle 3, die gegeneinander verschwenkbar ausgebildet sind. Die Dämpferwelle 3 ist drehbar in dem Gehäuse 12 über Wälzlager 44 gelagert. Die Dämpferwelle 3 ist hier insgesamt dreiteilig ausgebildet, wie mit Bezug auf die Fig. 20 erläutert wird.

Das Gehäuse 12 umfasst ein erstes Endteil 22 und ein zweites Endteil 24 am anderen Ende und ein dazwischen angeordnetes Mittelteil 23. An beiden Enden sind noch äußere Gehäuseteile 12a aufgenommen, an denen Schrauböffnungen ausgebildet sind. An dem radial äußeren Gehäuseteil 12a ist eine unrunde Koppelkontur 70 mit Ausnehmungen im Bereich des Endes der Bezugszeichenlinie ausgebildet. Mehrere über dem Umfang verteilt angeordnete Ausnehmungen bilden die unrunde Koppelkontur, womit eine drehfeste Verbindung mit weiteren Komponenten einer Hüft-, Knie, Fuß-, Ellbogen- oder z.B. Schulterprothese möglich ist.

In den beiden Endteilen 22 und 24 ist jeweils eine umlaufende elektrische Spule 9 aufgenommen, die zur Erzeugung des für die Dämpfung benötigten Magnetfeldes sorgt.

Das Magnetfeld ist wie in allen Ausführungsbeispielen steuerbar. Wie in allen Ausführungsbeispielen und Ausgestaltungen wird bei einem stärkeren Magnetfeld eine stärkere Dämpfung (Bremswirkung) erzeugt. Gleichzeitig wird durch das stärkere Magnetfeld auch eine bessere Abdichtung der Spalte 25, 26 und 27 (vergleiche die schematische Darstellung nach Fig. 13) erreicht. Umgekehrt wird in allen Ausführungsbeispielen und Ausgestaltungen durch ein schwächeres Magnetfeld eine schwächere Dämpfung (Bremswirkung) eingestellt. Gleichzeitig ist bei einem schwächeren Magnetfeld auch die Dichtwirkung an den Spalten 25 bis 27 geringer. Dadurch resultiert ein geringeres Grundmoment, welches ohne ein Magnetfeld wirkt. Die Dichtwirkung der Spalte 25 bis 27 ist ohne Magnetfeld gering. Dadurch kann ein weiter Einstellbereich zur Verfügung gestellt werden, was im Stand der Technik so nicht möglich ist. Das Verhältnis aus maximalem Drehmoment (bzw. maximaler Bremswirkung) zu minimalem Drehmoment (bzw. minimaler Bremswirkung) ist innerhalb des vorgesehenen Schwenkwinkels oder innerhalb des Arbeitsbereichs sehr groß und größer als im Stand der Technik.

Bei konventionellen Drehdämpfern von Prothesen ist hingegen das minimale Drehmoment schon groß, wenn ein hohes maximales Drehmoment erzeugt werden soll. Das liegt daran, dass die Dichtungen der Spalte so ausgeführt sein müssen, dass auch bei hohen wirkenden Drücken eine zuverlässige oder doch ausreichende Abdichtung sichergestellt wird. Umgekehrt wird bei Drehdämpfern von Prothesen, die im Leerlauf ein geringes Bremsmoment haben sollen, nur ein geringes maximales Drehmoment erreicht, da die Dichtungen so ausgelegt sind, dass nur wenig Reibung entsteht. Bei hohen wirksamen Drücken sorgt das für einen erheblichen Leckagestrom, der das maximal möglich Drehmoment stark begrenzt.

Der Innenraum des Drehdämpfers 1 stellt ein Dämpfervolumen zur Verfügung. In dem Gehäuse ist eine Verdrängereinrichtung 2 ausgebildet, die Trenneinheiten 4 und 5 umfasst. Die Trenneinheiten 4 und 5 trennen das Dämpfervolumen 60 in zwei oder mehr Kammern 61 und 62. Dabei ist die Trenneinheit 4 als Trennwand ausgebildet und fest mit dem Gehäuse 12 verbunden. Die Trenneinheit 5 ist ebenfalls als Trennwand oder als Schwenkflügel ausgebildet und ist fest mit der Dämpferwelle 3 verbunden. Vorzugsweise ist die Trenneinheit 5 einstückig mit der Dämpferwelle 3 ausgebildet. Das Dämpfervolumen 60 ist hier mit magnetorheologischen Fluid 6 gefüllt. Eine Abdichtung des Dämpfervolumens 60 nach außen erfolgt über eine Dichtung 28 in dem Gehäuseteil 22. Bei einer Schwenkbewegung verdrängen die Trenneinheiten 4 und 5 das in dem Dämpfervolumen enthaltene magnetorheologische Fluid (MRF), sodass das MRF zum Teil von der einen Kammer in die andere überströmt. Ein Verbindungskanal bzw. Ausgleichskanal 63 dient zum Druckausgleich zwischen den Kammern 61 und 61a. Ein entsprechender zweiter Verbindungskanal 63a (vgl. Fig. 20 dient zum Druckausgleich zwischen den Kammern 62 und 62a.

Am hinteren Ende ist in Fig. 18 noch ein Ventil 66 zu erkennen, durch welches ein kompressibles Fluid in die Ausgleichseinrichtung 30 eingefüllt wird. Insbesondere wird Stickstoff verwendet. Das Ventil 66 kann z. B. in einen eingeschraubten Abschluss oder Deckel integriert sein.

Am vorderen Ende ist in Fig. 18 außerhalb des Gehäuses 12 des Drehdämpfers 1 ein mechanischer Anschlag 64 zu sehen, der mechanisch den möglichen Schwenkbereich begrenzt, um die Schwenkflügel im Inneren vor Beschädigungen zu schützen.

Die Magnetfeldquelle 8 in dem Gehäuseteil 22 besteht hier aus elektrischen Spulen 9, die jeweils ringförmig ausgebildet sind und in dem Gehäuseteil 22 aufgenommen sind. Hier im Ausführungsbeispiel sind in beiden Endteilen elektrische Spulen 9 vorgesehen. Über eine Steuerung kann die Magnetfeldstärke vorgegeben werden.

In das Dämpfervolumen 60 ragen hier zwei Trenneinheiten 4 von dem Gehäuse radial nach innen. Die Trenneinheiten 4 bilden Trennwände und begrenzen so die mögliche Drehbewegung der Dämpferwelle 3, an der ebenfalls zwei Trenneinheiten 5 ausgebildet sind, die von der Dämpferwelle radial nach außen ragen. Durch Drehen der Dämpferwelle 3 werden die Trennwände 5 geschwenkt, die hier Schwenkflügel bilden. Die Kammern 61 und 61a werden entsprechend verkleinert (vgl. Fig. 19) oder wieder vergrößert.

In Fig. 19 sind noch vier Entlüftungsventile zu erkennen, die bei einem Prototypen eingesetzt wurden, um eine schnellere Befüllung und Entleerung zu erzielen und die gegebenenfalls auch nicht (alle) realisiert werden müssen.

Wie auch Fig. 20 zeigt, sind die elektrischen Spulen 9 hier im Ausführungsbeispiel radial relativ weit radial außen angeordnet und werden axial nach innen jeweils von einem magnetisch nicht oder nur schlecht leitenden Ring 20 begrenzt, der zu Formung des Magnetfeldverlaufes dient. Der Ring 20 weist insbesondere eine hohlzylindrische Form auf.

In dem vollständigen Längsschnitt nach Fig. 20 ist die Ausgleichseinrichtung 30 zu sehen, die hier im Inneren der Dämpferwelle 3 untergebracht ist. Die Ausgleichseinrichtung 30 umfasst ein mit MRF gefülltes Ausgleichsvolumen 29, welches durch einen beweglich angeordneten Trennkolben 34 von dem der Luftkammer 32 getrennt ist. Sowohl die Luftkammer 32 als auch der Trennkolben 34 und das Ausgleichsvolumen 29 sind innerhalb eines hohlzylindrischen Aufnahmeraumes 30a vollständig im Inneren der Dämpferwelle 3 untergebracht. Der Hohlzylinder 30a wird am axial äußeren Ende durch einen Abschluss mit dem Ventil 66 abgeschlossen. Diese Ausgestaltung erlaubt eine besonders kompakte und raumsparende Bauweise, bei der nur sehr wenige Teile von dem grundsätzlich im Wesentlichen zylindrisch ausgebildeten Drehdämpfer 1 abstehen. Das erhöht die Einbau- und Verwendungsmöglichkeiten derartiger Protheseneinrichtungen 100.

Die Ausgleichseinrichtung 30 ist in Fig. 18 bis 20 über nicht dargestellte Kanäle mit dem Kanal 72 verbunden, der hier durch einen Verschluss 71 verschlossen ist. Dadurch ist es optional möglich, eine externe Ausgleichseinrichtung 30 anzukoppeln und im Inneren einen Einsatz einzusetzen, um das Volumen des Hohlzylinders 30a weitgehend auszufüllen. Dadurch kann z. B. ein besonders großer Temperaturbereich ausgeglichen werden. Es ist auch möglich, darüber eine besonders lange Laufzeit zu gewährleisten, auch wenn etwas Leckage auftritt.

In Fig. 20 ist die hier dreiteilige Dämpferwelle 3 gut zu erkennen, die hier aus der Hohlwelle 3a, der Anschlusswelle 3b und dem Ansatz 3c besteht. Die drei Teile sind drehfest miteinander gekoppelt. Möglich ist es auch, die Dämpferwelle 3 zweiteilig oder auch nur einteilig auszubilden.

Fig. 21 zeigt eine Variante des Ausführungsbeispiels nach den Figuren 18 bis 20, wobei hier eine externe Ausgleichseinrichtung 30 angekoppelt ist. Die weiteren Bauteile können identisch sein. Praktisch kann an dem Drehdämpfer 1 nach Fig. 18 der Verschluss 71 entfernt werden und die dargestellte außenliegende Ausgleichseinrichtung angeschraubt werden. Im Inneren ist eine Luft- bzw. Fluidkammer 32 ausgebildet, die durch einen Trennkolben 34 von dem mit MRF gefülltem Ausgleichsvolumen 29 getrennt ist.

Im Inneren ist in dem Hohlzylinder 30a ein Einsatz 67 untergebracht, um das Volumen auszufüllen.

Im Ausführungsbeispiel nach Fig. 21 sind noch zwei Winkelsensoren 68 und 69 angebracht. Hier misst ein Winkelsensor 68 mit geringerer Genauigkeit die absolute Winkelposition und der Winkelsensor 69 mit höherer Genauigkeit eine relative Winkelposition. Dadurch kann ein hochgenaues Sensorsystem zur Verfügung gestellt werden, welches robust und zuverlässig und dennoch hochgenau arbeitet.

Insgesamt wird eine vorteilhafte Protheseneinrichtung 100 mit einem Drehdämpfer 1 zur Verfügung gestellt. Um die temperaturbedingte Volumenausdehnung der MR-Flüssigkeit (MRF) und der angrenzenden Bauteilen kompensieren zu können, ist es sinnvoll, ein adäquates Ausgleichsvolumen vorzusehen.

In einem konkreten Fall werden ca. 50ml MRF pro Einzelaktor bzw. Drehdämpfer benötigt und somit ca. 150ml für das Gesamtsystem. Als Vorspannelement dient vorzugsweise ein Stickstoffvolumen, welches insbesondere mit ca. 75bar vorgespannt wird.

Verwendet wurde in diesem Beispiel ein Spulendraht mit effektivem Querschnitt von 0,315mm². Die Windungszahl von 400 ergab ein Füllfaktor von ca. 65% bei 16 Ohm Widerstand. Mit einem größeren Drahtdurchmesser kann noch eine größere Spulengeschwindigkeit erreicht werden.

Vorzugsweise wird ein Axialspiel der Trennwände bzw. Schwenkflügel eingestellt. Für eine einwandfreie Funktion des Aktors ist es vorteilhaft, die axiale Position des Schwenkflügels 5 zum Gehäuse auszumitteln und einzustellen. Dazu können z. B. Gewindestellringe verwendet werden, die mit einer Messuhr in die Mittellage gebracht werden.

In einem konkreten Fall erfolgte eine Befüllung mit MRF, wobei (knapp) 75 ml MRF eingefüllt wurden. Zum Einfüllen kann das MRF über das Ausgleichsvolumen eingefüllt werden. Unter wechselseitigem Bewegen des Schwenkflügels kann das MRF innerhalb der Kammern 61, 62 (Druckraum) verteilt werden und es können Lufteinschlüsse nach oben befördert werden. Anschließend kann das System mit Stickstoff (ca. 5bar) vorgespannt werden. Danach können die Entlüftungsschrauben 65 an der Außenseite des Gehäuses 12 geöffnet werden, um die eingeschlossene Luft entweichen zu lassen. Abschließend wurde die Stickstoffkammer 32 auf 30bar für erste Tests am Prüfstand vorgespannt.

Als Optimierungsmaßnahme kann der Aktor der Prothese auch in eine Unterdruckumgebung gebracht werden, um möglich Lufteinschlüsse besser evakuieren zu können.

Es werden hohe Drücke ohne mechanische Dichtung erreicht. Die Protheseneinrichtung 100 mit dem Drehdämpfer 1 ist kostengünstig herstellbar, robust und langlebig.

Es wurden bei diesem konkreten Beispiel >210Nm Bremsmoment am Prüfstand erreicht. Die Einheit baut kleiner, leichter und kostengünstiger als im Stand der Technik.

Schaltzeiten von <30ms sind möglich und konnten nachgewiesen (Vollastsprung) werden.

Das Bremsmoment kann beliebig variiert werden. Hierfür sind keine mechanisch bewegten Teile erforderlich. Die Steuerung erfolgt einfach nur über Strom- bzw. Magnetfeldvariation.

Ein erheblicher Vorteil ergibt sich aufgrund fehlender mechanischer Dichtungen. Dadurch wird ein sehr niedriges Grundmoment kleiner 0,5Nm erreicht. Das wird daurch erreicht, dass nicht nur das Bremsmoment, sondern gleichzeitig auch die Dichtungswirkung der Dichtungen gesteuert wird. Insgesamt ergibt sich ein sehr niederer Leistungsbedarf von im Beispiel wenigen Watt.

Der Drehdämpfer 1 kann in verschiedenen Protheseneinrichtungen 100 eingesetzt werden. Eine Anwendung als Hüft(teil)prothese, als Knieprothese, als Fuß, Ellbogen, Schulter(teil)prothese ist möglich. Darin kann ein entsprechend angepasster Drehdämpfer 1 eingebaut sein. Dabei kann die Dimensionierung an die gewünschten aufzubringenden Kräfte und Momente angepasst werden. Eine entsprechende Skalierung ist möglich.

In allen Ausgestaltungen kann eine Protheseneinrichtung 100 als Prothese, Orthese oder auch als Exoskelett ausgebildet sein.

**Bezugszeichenliste:**

| | | | |
|---|---|---|---|
| 1 | Drehdämpfer | 33 | Dichtung |
| 2 | Verdrängereinrichtung | 34 | Trennkolben |
| 3 | Dämpferwelle | 35 | Deckel |
| 3a | Hohlwelle | 36 | Kanal |
| 3b | Anschlusswelle | 37 | Energiespeicher |
| 4 | Trenneinheit, Trennwand | 39 | Dauermagnet |
| 5 | Trenneinheit, Trennwand | 40 | Sensoreinrichtung |
| 6 | MRF | 41 | Abstand |
| 7 | Steuereinrichtung | 42 | Dichtung von 23 |
| 8 | Magnetfeldquelle | 43 | Zwischenraum |
| 9 | elektrische Spule | 44 | Lager |
| 10 | Magnetfeld | 45 | Belastungssensor |
| 11 | Anschluss (an 12) | 46 | Arm |
| 12 | Gehäuse von 2 | 47 | Feder, Torsionsstab |
| 12a | äußeres Gehäuseteil | 48 | Sensorleitung |
| 13 | Anschluss (an 3) | 52 | Ventileinheit |
| 14 | Isolator | 53 | Bewegungsrichtung |
| 15 | Hydraulikleitung | 54 | Druckspeicher |
| 16 | Stromanschluss | 55 | Pfeilrichtung |
| 17 | Dämpfungskanal | 60 | Dämpfervolumen |
| 19 | Achse von 3, 9 | 61 | Kammer |
| 20 | Ring in 12 | 62 | Kammer |
| 22 | erster Endbereich | 63 | Verbindungs kanal |
| 23 | Mittelbereich | 63a | zweiter Verbindungskanal |
| 24 | zweiter Endbereich | 64 | mechanischer Anschlag |
| 25 | Spalt, Axialspalt | 65 | Entlüftungsschraube |
| 26 | Spalt, Radialspalt | 66 | Stickstoffventil |
| 27 | Spalt, Radialspalt | 67 | Einsatz |
| 28 | Dichtung an 3 | 68 | Sensor |
| 29 | Ausgleichsvolumen | 69 | Sensor |
| 30 | Ausgleichseinrichtung | 70 | unrunde Koppelkontur |
| 30a | Hohlzylinder | 71 | Verschluss |
| 31 | Ventileinheit | 72 | Kanal |
| 31a | Ventilkopf | 100 | Protheseneinrichtung |
| 32 | Luftkammer | | |

## Patentansprüche

1. Protheseneinrichtung (100) mit einem Drehdämpfer (1) mit einem Gehäuse (12), einer drehbar daran aufgenommenen Dämpferwelle (3), einer Verdrängereinrichtung (2) in dem Gehäuse (12), und mit wenigstens einer Magnetfeldquelle (8), wobei die Verdrängereinrichtung (2) ein Dämpfervolumen (60) mit einem magnetorheologischen Fluid (6) als Arbeitsfluid aufweist und damit betreibbar ist, um eine Dämpfung der Drehbewegung der Dämpferwelle (3) relativ zu dem Gehäuse (12) zu beeinflussen, wobei die Verdrängereinrichtung (2) wenigstens zwei Trenneinheiten (4, 5) umfasst, mit denen das Dämpfervolumen (60) in wenigstens zwei variable Kammern (61, 62) unterteilt wird, wobei wenigstens eine der Trenneinheiten (4, 5) eine mit dem Gehäuse (12) verbundene Trennwand (4) umfasst,
und wobei wenigstens eine der Trenneinheiten (5) eine mit der Dämpferwelle (3) verbundene Trennwand (5) umfasst,
wobei zwischen der mit dem Gehäuse (12) verbundenen Trenneinheit (4) und der Dämpferwelle (3) in radialer Richtung ein Spaltabschnitt (26) ausgebildet ist,
und wobei zwischen der mit der Dämpferwelle (3) verbundenen Trenneinheit (5) und dem Gehäuse (12) in radialer Richtung ein Spaltabschnitt (27) ausgebildet ist,
und wobei zwischen der mit der Dämpferwelle (3) verbundenen Trenneinheit (5) und dem Gehäuse (12) in axialer Richtung wenigstens ein Spaltabschnitt (25) ausgebildet ist, wobei wenigstens ein wesentlicher Teil des Magnetfeldes (10) der Magnetfeldquelle (8) wenigstens zwei der angeführten Spaltabschnitte (25-27) durchtritt.

2. Protheseneinrichtung (100) nach dem vorhergehenden Anspruch, wobei wenigstens einer der Spaltabschnitte (27) als Dämpfungsspalt und wenigstens einer der Spaltabschnitte (25, 26) als Dichtspalt ausgebildet ist und wobei wenigstens ein Dämpfungsspalt (25) eine größere Spalthöhe als ein Dichtspalt aufweist.

3. Protheseneinrichtung (100) nach einem der vorhergehenden Ansprüche, wobei die Magnetfeldquelle (8) eine elektrische Spule (9) oder wobei wenigstens zwei elektrische Spulen (9) umfasst.

4. Protheseneinrichtung (100) nach einem der vorhergehenden Ansprüche, wobei an beiden axialen Enden der mit der Dämpferwelle (3) verbundenen Trennwand (5) jeweils ein axialer Spaltabschnitt (25) zwischen dem Gehäuse und der Trennwand (5) ausgebildet ist und wobei ein wesentlicher Teil des Magnetfeldes (10) der Magnetfeldquelle (8) durch beide axiale Spaltabschnitte (25) zwischen dem Gehäuse (12) und der Trennwand (5) durchtritt und eine Dichtung der axialen Spaltabschnitte (25) bewirkt, wobei das Magnetfeld (10) insbesondere quer zu wenigstens einem der Spaltabschnitte (25-27) verläuft.

5. Protheseneinrichtung (100) nach einem der vorhergehenden Ansprüche, wobei zumindest ein radialer Spaltabschnitt (27) als Dämpfungskanal (17) ausgebildet ist und radial zwischen der Trenneinheit (5) und dem Gehäuse (12) angeordnet ist und/oder wobei zumindest ein axialer Spaltabschnitt (25) als Dämpfungskanal (17) ausgebildet ist und axial zwischen der Trenneinheit (5) und dem Gehäuse (12) angeordnet ist, wobei insbesondere wenigstens ein wesentlicher Teil des Magnetfeldes (10) der Magnetfeldquelle (8) durch den Dämpfungskanal (17) durchtritt.

6. Protheseneinrichtung (100) nach einem der vorhergehenden Ansprüche, wobei das Gehäuse (12) ein erstes und ein zweites Endteil (22, 24) und dazwischen ein Mittelteil (23) umfasst, wobei in wenigstens einem der beiden Endteile (22, 24) und insbesondere in beiden Endteilen (22, 24) eine elektrische Spule (9) aufgenommen ist, wobei eine Achse (19) der Spule (9) insbesondere im Wesentlichen parallel zur Dämpferwelle (3) ausgerichtet ist.

7. Protheseneinrichtung (100) nach einem der vorhergehenden Ansprüche, wobei das Gehäuse (12) wenigstens zu einem wesentlichen Teil aus einem magnetisch leitenden Material mit einer relativen Permeabilität größer 100 besteht und/oder wobei axial benachbart zu der elektrischen Spule (9) in dem Gehäuse (12) ein Ring (20) angeordnet ist.

8. Protheseneinrichtung (100) nach einem der vorhergehenden Ansprüche, wobei das magnetorheologische Fluid (6) durch eine relative Schwenkbewegung der Dämpferwelle und des Gehäuses durch wenigstens einen Spaltabschnitt (25-27) von einer Kammer (61, 62) in die andere Kammer (62, 61) gefördert wird, wobei insbesondere der Dämpfungskreislauf des magnetorheologischen Fluids (6) vollständig innerhalb des Gehäuses (12) angeordnet ist.

9. Protheseneinrichtung (100) nach einem der vorhergehenden Ansprüche, wobei an der Dämpferwelle (3) zwei oder mehr über dem Umfang verteilt angeordnete Trenneinheiten (5) ausgebildet sind und wobei an dem Gehäuse (12) zwei oder mehr über dem Umfang verteilt angeordnete Trenneinheiten (4) ausgebildet sind, wobei insbesondere sich gegenüberliegende Kammern (61, 61a; 62, 62a) durch wenigstens einen Verbindungskanal (63) verbunden sind.

10. Protheseneinrichtung (100) nach einem der vorhergehenden Ansprüche, wobei eine Ausgleichseinrichtung (30) mit einem Ausgleichsvolumen (29) vorgesehen ist und wobei das Ausgleichsvolumen (29) über eine Ventileinheit (31) mit den beiden Kammern verbunden ist, wobei die Ventileinheit dazu ausgebildet ist, eine Verbindung zwischen dem Ausgleichsvolumen (29) und einer Niederdruckkammer (62; 61) herzustellen und eine Verbindung zwischen dem Ausgleichsvolumen (29) und der Hochdruckkammer (61; 62) zu blockieren.

11. Protheseneinrichtung (100) nach einem der vorhergehenden Ansprüche, **gekennzeichnet durch** einen Temperatursensor (34) zur Erfassung einer Temperatur des magnetorheologischen Fluids (6) und/oder durch einen Winkelsensor (40) zur Erfassung eines Maßes für eine Winkelposition der Dämpferwelle (3) und/oder durch einen Belastungssensor zur Erfassung eines Kennwertes für ein Drehmoment an der Dämpferwelle (3).

12. Protheseneinrichtung (100) nach einem der vorhergehenden Ansprüche, wobei wenigstens eine Sensoreinrichtung (40) umfassend wenigstens einen Positions- und/oder Abstandssensor zur Erfassung einer Position und/oder eines Abstands (41) von umgebenden Objekten vorgesehen ist, wobei die Steuereinrichtung (7) dazu ausgebildet und eingerichtet ist, den Drehdämpfer in Abhängigkeit von den Sensordaten der Sensoreinrichtung zu steuern, insbesondere umfassend eine Steuereinrichtung (7) und eine Mehrzahl miteinander verbundener Drehdämpfer (1).

13. Protheseneinrichtung (100) nach einem der vorhergehenden Ansprüche, ausgebildet als Prothese, Orthese oder als Exoskelett.

14. Protheseneinrichtung (100) mit einem Drehdämpfer (1) mit einem Gehäuse (12), wenigstens einer Magnetfeldquelle und einem mit einem magnetorheologischen Fluid ausgerüsteten Dämpfervolumen, welches durch wenigstens eine mit einer Dämpferwelle (3) verbundene Trenneinheit (5) in wenigstens zwei Kammern (61, 62) unterteilt wird, wobei zwischen der Trenneinheit (5) und dem Gehäuse (22-24) Spaltabschnitte (25-27) ausgebildet sind, wobei das Gehäuse (22-24), die Magnetfeldquelle (8) und die Trenneinheit (5) dazu ausgebildet und eingerichtet sind, dass ein Magnetfeld der Magnetfeldquelle (8) die wesentlichen Spaltabschnitte (25-27) zwischen der Trenneinheit (5) und dem Gehäuse (22-24) durchflutet.

15. Verfahren zum Dämpfen von Bewegungen einer Protheseneinrichtung (100) mit einem Drehdämpfer, wobei der Drehdämpfer wenigstens eine Magnetfeldquelle und eine mit einem magnetorheologischen Fluid ausgerüstetes Dämpfervolumen aufweist, welches durch wenigstens eine mit einer Dämpferwelle (3) verbundene Trenneinheit (5) in wenigstens zwei Kammern (61, 62) unterteilt wird, wobei zwischen der Trenneinheit (5) und dem Gehäuse (22-24) Spaltabschnitte (25-27) ausgebildet sind, wobei mit einem Magnetfeld der Magnetfeldquelle (8) die wesentlichen Spaltabschnitte (25-27) zwischen der Trenneinheit (5) und dem Gehäuse (22-24) durchflutet werden, um die Dämpfung zu beeinflussen.

## Claims

1. Prosthesis device (100) with a rotary damper (1) comprising a housing (12), a damper shaft (3) rotatably accommodated thereat, a displacing device (2) in the housing (12), and comprising at least one magnetic field source (8), wherein the displacing device (2) comprises a damper volume (60) with magnetorheological fluid (6) as a working fluid by means of which it can operate to influence the damping of the rotary motion of the damper shaft (3) relative to the housing (12), wherein the displacing device (2) comprises at least two partition units (4, 5) which subdivide the damper volume (60) into at least two variable chambers (61, 62), wherein at least one of the partition units (4, 5) comprises a partition wall (4) connected with the housing (12),
and wherein at least one of the partition units (5) comprises a partition wall (5) connected with the damper shaft (3), wherein a gap section (26) is configured in the radial direction between the partition unit (4) connected with the housing (12) and the damper shaft (3),
and wherein a gap section (27) is configured in the radial direction between the partition unit (5) connected with the damper shaft (3) and the housing (12),
and wherein at least one gap section (25) is configured in the axial direction between the partition unit (5) connected with the damper shaft (3) and the housing (12),
wherein at least a substantial part of the magnetic field (10) of the magnetic field source (8) passes through at least two of the indicated gap sections (25-27).

2. The prosthesis device (100) according to the preceding claim, wherein at least one of the gap sections (27) is configured as a damping gap, and at least one of the gap sections (25, 26), as a sealing gap, and wherein at least one damping gap (25) shows a larger gap height than does a sealing gap.

3. The prosthesis device (100) according to any of the preceding claims, wherein the magnetic field source (8) comprises one electric coil (9), or wherein it comprises at least two electric coils (9).

4. The prosthesis device (100) according to any of the preceding claims, wherein both of the axial ends of the partition wall (5) connected with the damper shaft (3) are each configured with an axial gap section (25) between the housing and the partition wall (5), and wherein a substantial part of the magnetic field (10) of the magnetic field source (8) passes through both axial gap sections (25) between the housing (12) and the partition wall (5) and provides for sealing the axial gap sections (25), wherein the magnetic field (10) extends in particular transverse to at least one of the gap sections (25-27).

5. The prosthesis device (100) according to any of the preceding claims, wherein at least one radial gap section (27) is configured as a damping duct (17) and is disposed radially between the partition unit (5) and the housing (12), and/or wherein at least one axial gap section (25) is configured as a damping duct (17) and is disposed axially between the partition unit (5) and the housing (12), wherein in particular at least a substantial part of the magnetic field (10) of the magnetic field source (8) passes through the damping duct (17).

6. The prosthesis device (100) according to any of the preceding claims, wherein the housing (12) comprises a first and a second end part (22, 24) and in-between, a center part (23), wherein at least one of the two end parts (22, 24) and in particular both of the end parts (22, 24) accommodate an electric coil (9), wherein the axis (19) of the coil (9) is in particular oriented substantially in parallel to the damper shaft (3).

7. The prosthesis device (100) according to any of the preceding claims, wherein the housing (12) consists at least substantially of a magnetically conductive material showing relative permeability of above 100, and/or wherein a ring (20) is disposed axially adjacent to the electric coil (9) in the housing (12).

8. The prosthesis device (100) according to any of the preceding claims, wherein the magnetorheological fluid (6) is conveyed by way of a relative pivoting motion of the damper shaft and the housing through at least one gap section (25-27) from one chamber (61, 62) into the other chamber (62, 61), wherein in particular the damping circuit of the magnetorheological fluid (6) is disposed completely inside the housing (12).

9. The prosthesis device (100) according to any of the preceding claims, wherein two or more partition units (5) are configured disposed on the damper shaft (3) distributed over the circumference, and wherein two or more partition units (4) are configured disposed on the housing (12) distributed over the circumference, wherein in particular opposite chambers (61, 61a; 62, 62a) are connected through at least one connection duct (63) .

10. The prosthesis device (100) according to any of the preceding claims, wherein an equalizing device (30) with an equalizing volume (29) is provided, and wherein the equalizing volume (29) is connected with the two chambers through a valve unit (31), wherein the valve unit is configured to establish a connection between the equalizing volume (29) and a low pressure chamber (62; 61) and to block a connection between the equalizing volume (29) and the high pressure chamber (61; 62).

11. The prosthesis device (100) according to any of the preceding claims, **characterized by** a temperature sensor (34) for capturing the temperature of the magnetorheological fluid (6), and/or by an angle sensor (40) for capturing a measure for an angular position of the damper shaft (3), and/or by a load sensor for capturing a characteristic value of a rotational force on the damper shaft (3).

12. The prosthesis device (100) according to any of the preceding claims, wherein at least one sensor device (40) is provided, comprising at least one position- and/or distance sensor for capturing the position and/or distance (41) from surrounding objects, wherein the control device (7) is configured and set up to control the rotary damper in dependence on the sensor data from the sensor device, in particular comprising a control device (7) and a plurality of interconnected rotary dampers (1).

13. The prosthesis device (100) according to any of the preceding claims, configured as a prosthesis, an orthosis, or an exoskeleton.

14. Prosthesis device (100) with a rotary damper (1), comprising a housing (12), at least one magnetic field source and a damper volume provided with magnetorheological fluid, which is subdivided into at least two chambers (61, 62) by at least one partition unit (5) connected with a damper shaft (3), wherein gap sections (25-27) are formed between the partition unit (5) and the housing (22-24),
wherein the housing (22-24), the magnetic field source (8) and the partition unit (5) are configured and set up for a magnetic field of the magnetic field source (8) to flow through the significant gap sections (25-27) between the partition unit (5) and the housing (22-24).

15. Method for damping movements of a prosthesis device (100) by means of a rotary damper, wherein the rotary damper comprises at least one magnetic field source and a damper volume provided with magnetorheological fluid, and is subdivided into at least two chambers (61, 62) by at least one partition unit (5) connected with a damper shaft (3), wherein gap sections (25-27) are configured between the partition unit (5) and the housing (22-24),
wherein a magnetic field of the magnetic field source (8) flows through the significant gap sections (25-27) between the partition unit (5) and the housing (22-24) to influence the damping.

## Revendications

1. Dispositif prothétique (100) avec un amortisseur rotatif (1) comprenant un boîtier (12), un arbre d'amortisseur (3) reçu en rotation sur celui-ci, un dispositif de déplacement (2) à l'intérieur du boîtier (12), et avec au moins une source de champ magnétique (8), dans lequel ledit dispositif de déplacement (2) présente un volume d'amortisseur (60) ayant un fluide magnétorhéologique (6) en tant que fluide de travail et peut être actionné avec celui-ci afin d'influencer un amortissement du mouvement de rotation de l'arbre d'amortisseur (3) par rapport au boîtier (12), dans lequel le dispositif de déplacement (2) comprend au moins deux unités de séparation (4, 5) par lesquelles le volume d'amortisseur (60) est divisé en au moins deux chambres (61, 62) variables, dans lequel l'une au moins des unités de séparation (4, 5) comprend une cloison (4) reliée au boîtier (12), et dans lequel l'une au moins des unités de séparation (5) comprend une cloison (5) reliée à l'arbre d'amortisseur (3),
dans lequel une section de fente (26) est formée dans la direction radiale entre l'unité de séparation (4) reliée au boîtier (12) et l'arbre d'amortisseur (3),
dans lequel une section de fente (27) est formée dans la direction radiale entre l'unité de séparation (5) reliée à l'arbre d'amortisseur (3) et le boîtier (12),
et dans lequel au moins une section de fente (25) est formée dans la direction axiale entre l'unité de séparation (5) reliée à l'arbre d'amortisseur (3) et le boîtier (12),
dans lequel au moins une partie substantielle du champ magnétique (10) de la source de champ magnétique (8) passe à travers au moins deux des sections de fente (25 à 27) mentionnées.

2. Dispositif prothétique (100) selon la revendication précédente, dans lequel l'une au moins des sections de fente (27) est conçue en tant que fente d'amortissement et l'une au moins des sections de fente (25, 26) est conçue en tant que fente d'étanchéité et dans lequel au moins une fente d'amortissement (25) présente une hauteur de fente supérieure à celle d'une fente d'étanchéité.

3. Dispositif prothétique (100) selon l'une quelconque des revendications précédentes, dans lequel la source de champ magnétique (8) comprend une bobine électrique (9) ou au moins deux bobines électriques (9).

4. Dispositif prothétique (100) selon l'une quelconque des revendications précédentes, dans lequel respectivement une section de fente (25) axiale est formée entre le boîtier et la cloison (5) aux deux extrémités axiales de la cloison (5) reliée à l'arbre d'amortisseur (3), et dans lequel une partie substantielle du champ magnétique (10) de la source de champ magnétique (8) passe à travers les deux sections de fente (25) axiales entre le boîtier (12) et la cloison (5) et provoque une étanchéité des sections de fente (25) axiales, dans lequel le champ magnétique (10) s'étend en particulier transversalement à au moins l'une des sections de fente (25-27).

5. Dispositif prothétique (100) selon l'une quelconque des revendications précédentes, dans lequel au moins une section de fente (27) radiale est réalisée en tant que canal d'amortissement (17) et est disposée radialement entre l'unité de séparation (5) et le boîtier (12) et/ou dans lequel au moins une section de fente (25) axiale est conçue en tant que canal d'amortissement (17) et est disposée axialement entre l'unité de séparation (5) et le boîtier (12), dans lequel, en particulier, au moins une partie substantielle du champ magnétique (10) de la source de champ magnétique (8) passe à travers le canal d'amortissement (17).

6. Dispositif prothétique (100) selon l'une quelconque des revendications précédentes, dans lequel le boîtier (12) comprend une première et une deuxième partie d'extrémité (22, 24) ainsi qu'une partie de milieu (23) entre celles-ci, dans lequel une bobine électrique (9) est reçue dans au moins l'une des deux parties d'extrémité (22, 24) et en particulier dans les deux parties d'extrémité (22, 24), dans lequel un axe (19) de la bobine (9) est orienté en particulier pour l'essentiel parallèlement à l'arbre d'amortisseur (3).

7. Dispositif prothétique (100) selon l'une quelconque des revendications précédentes, dans lequel le boîtier (12) est constitué, au moins pour une partie substantielle, d'un matériau magnétiquement conducteur ayant une perméabilité relative supérieure à 100 et/ou dans lequel une bague (20) est disposée de manière axialement voisine de la bobine électrique (9) dans le boîtier (12).

8. Dispositif prothétique (100) selon l'une quelconque des revendications précédentes, dans lequel le fluide magnétorhéologique (6) est transporté par un mouvement de pivotement relatif de l'arbre d'amortisseur et du boîtier, à travers au moins une section de fente (25-27) depuis une chambre (61, 62) dans l'autre chambre (62, 61), dans lequel, en particulier, le circuit d'amortissement du fluide magnétorhéologique (6) est disposé complètement à l'intérieur du boîtier (12).

9. Dispositif prothétique (100) selon l'une quelconque des revendications précédentes, dans lequel deux ou plusieurs unités de séparation (5) réparties sur la circonférence sont réalisées sur l'arbre d'amortisseur (3), et dans lequel deux ou plusieurs unités de séparation (4) réparties sur la circonférence sont réalisées sur le boîtier (12), dans lequel, en particulier, des chambres (61, 61a; 62, 62a) se faisant face sont reliées par au moins un canal de liaison (63).

10. Dispositif prothétique (100) selon l'une quelconque des revendications précédentes, dans lequel un dispositif de compensation (30) ayant un volume de compensation (29) est prévu et dans lequel le volume de compensation (29) est relié aux deux chambres par une unité de vanne (31), dans lequel ladite unité de vanne est conçue pour établir une connexion entre le volume de compensation (29) et une chambre basse pression (62; 61) et pour bloquer une connexion entre le volume de compensation (29) et la chambre haute pression (61; 62).

11. Dispositif prothétique (100) selon l'une quelconque des revendications précédentes, **caractérisé par** un capteur de température (34) destiné à détecter une température du fluide magnétorhéologique (6) et/ou par un capteur d'angle (40) destiné à détecter une mesure pour une position angulaire de l'arbre d'amortisseur (3) et/ou par un capteur de charge destiné à détecter une valeur caractéristique pour un couple sur l'arbre d'amortisseur (3).

12. Dispositif prothétique (100) selon l'une quelconque des revendications précédentes, dans lequel au moins un dispositif capteur (40) comprenant au moins un capteur de position et/ou de distance destiné à détecter une position et/ou une distance (41) d'objets environnants est prévu, dans lequel le dispositif de commande (7) est conçu et configuré pour commander l'amortisseur rotatif en fonction des données de capteur du dispositif capteur, en particulier comprenant un dispositif de commande (7) et une pluralité d'amortisseurs rotatifs (1) reliés entre eux.

13. Dispositif prothétique (100) selon l'une quelconque des revendications précédentes, réalisé en tant que prothèse, orthèse ou en tant qu'exosquelette.

14. Dispositif prothétique (100) avec un amortisseur rotatif (1) comprenant un boîtier (12), au moins une source de champ magnétique et un volume d'amortisseur équipé d'un fluide magnétorhéologique, qui est divisé en au moins deux chambres (61, 62) par au moins une unité de séparation (5) reliée à un arbre d'amortisseur (3), dans lequel des sections de fente (25-27) sont formées entre l'unité de séparation (5) et le boîtier (22-24),
dans lequel le boîtier (22-24), la source de champ magnétique (8) et l'unité de séparation (5) sont conçus et configurés de telle sorte qu'un champ magnétique de la source de champ magnétique (8) circule à travers les sections de fente (25-27) essentielles entre l'unité de séparation (5) et le boîtier (22-24).

15. Procédé destiné à amortir des mouvements d'un dispositif prothétique (100) avec un amortisseur rotatif (1), dans lequel ledit amortisseur rotatif comprend au moins une source de champ magnétique et un volume d'amortisseur équipé d'un fluide magnétorhéologique, qui est divisé en au moins deux chambres (61, 62) par au moins une unité de séparation (5) reliée à un arbre d'amortisseur (3), dans lequel des sections de fente (25-27) sont formées entre l'unité de séparation (5) et le boîtier (22-24),
dans lequel un champ magnétique de la source de champ magnétique (8) circule à travers les sections de fente (25-27) essentielles entre l'unité de séparation (5) et le boîtier (22-24), afin d'influencer l'amortissement.
